# EUROPEAN PATENT APPLICATION

(11) **EP 4 385 524 A1**
(43) Date of publication of application: **19.06.2024**
(21) Application number: 22856165.0
(22) Date of filing: 09.08.2022
(51) Int. Cl.: A61K 48/00, A61K 31/713, A61P 35/00, C12Q 1/6886, G01N 33/574, G01N 33/68, G01N 33/50

(54) **COMPOSITION FOR PREVENTING OR TREATING CANCER COMPRISING MSH2 OR MSH6 INHIBITOR AS ACTIVE INGREDIENT**

(30) Priority: 09.08.2021 KR 20210104655; 09.08.2021 KR 20210104656; 10.06.2022 KR 20220070947; 10.06.2022 KR 20220070948
(71) Applicant: Neornat, Seocho-gu Seoul 06591 (KR)
(72) Inventor: NAM, Suk-Woo, Seoul 06600 (KR); NA, Min Jeong, Seoul 06738 (KR)
(74) Representative: AWA Sweden AB
(86) International application number: PCT/KR2022/011831
(87) International publication number: WO 2023/018168

(57) **Abstract**

The present invention relates to a composition for preventing or treating cancer, comprising an MSH2 or MSH6 inhibitor as an active ingredient. The MSH2 or MSH6 inhibitor according to the present invention was confirmed to: inhibit tumor cell growth and proliferation in various carcinoma cells, including hepatocellular carcinoma, and in particular, inhibit migration, invasion, and tumorigenic activity in hepatocellular carcinoma; and increase DNMT1 expression and reduce KLF4 expression, when MSH2 or MSH6 expression was increased, and thus can be used as a pharmaceutical composition for the prevention and treatment of cancer.

## Description

### [Technical Field]

The present invention relates to a composition for preventing or treating cancer, including an MSH2 or MSH6 inhibitor as an active ingredient.

### [Background Art]

MutS homolog2 (MSH2) is well-known as a DNA mismatch repair protein. In hereditary nonpolyposis colorectal cancer (HNPCC, Lynch syndrome), at least 40% of MSH2 mutations are identified, and known as the main cause of HNPCC together with MLH1 mutations. When these mutations occur, mismatches in various genes are not corrected and are accumulated in a mutated state and play an important role in a carcinogenesis process.

MutS homolog6 (MSH6, GTBP) is well-known as a DNA mismatch repair protein. MSH6 forms a hetero dimer with MSH2, which serves as a factor that recognizes mismatch sites during a DNA replication process and initiates edition.

MSH2 forms a hetero dimer with MSH6, which serves as a factor that recognizes mismatch sites during a DNA replication process and initiates edition.

Histone deacetylases (HDACs) may be often attached to gene promoters by corepressors or multi-protein transcriptional complexes and regulate transcription through chromatin modification rather than directly binding to DNA. There are 18 encrypted human HDACs, which are classified into Class I (HDACs 1, 2, 3, and 8), Class II (HDACs 4, 5, 6, 7, 9, and 10), Class III (SIRT 1-7), and Class IV (HDAC11) enzymes. It is known that both histone acetyltransferases and HDACs are involved in cell proliferation, differentiation, and cell cycle regulation. In addition, it has been reported that pathological activity and deregulation of HDACs may cause many diseases, such as cancer, immune diseases, and muscular dystrophy. HDAC6 is a member of Class IIb family of HDACs, acts as cytoplasmic deacetylase associated with microtubules (MTs), and deacetylates α-tubulin.

DNA (cytosine-5)-methyltransferase 1 (DNMT1) is an enzyme that transfers a methyl group to a specific CpG structure in DNA. In the Cancer Genome Atlas project, the DNMT family is one of 127 mutated genes identified in cancer. It has been studied that DNMT1 is overexpressed in various cancers to be a therapeutic target.

Kruppel-like factor 4 (KLF4) is a zinc finger protein that may act in both directions of activating or inhibiting transcription of genes related to cell proliferation, differentiation, and death. KLF4 is involved in the growth inhibition and differentiation of cells after cell division in mucosal epithelial cells to be mainly overexpressed in the nucleus of growth-inhibited cells, and activates a cyclindependent kinase inhibitor, p21WAF1/Cip1 in a p53-dependent manner to inhibit the cell cycle in G1/S.

### [Disclosure]

### [Technical Problem]

An object of the present invention is to provide a pharmaceutical composition for preventing or treating cancer including as an active ingredient an inhibitor for an MutS homolog2 (MSH2) gene or protein; or an inhibitor for an MutS homolog6 (MSH6) gene or protein.

Another object of the present invention is to provide a method for treating liver cancer including administering the pharmaceutical composition to a subject.

Yet another object of the present invention is to provide a composition for diagnosing cancer including a preparation for measuring the level of the MSH2 or MSH6 gene, its protein, or a peptide constituting the protein.

Yet another object of the present invention is to provide a method for screening a substance for prevention or treatment of cancer.

### [Technical Solution]

One aspect of the present invention provides a pharmaceutical composition for preventing or treating cancer including as an active ingredient an inhibitor for an MutS homolog2 (MSH2) gene or protein; or an inhibitor for an MutS homolog6 (MSH6) gene or protein.

Another aspect of the present invention provides a method for treating liver cancer including administering the pharmaceutical composition to a subject.

Yet another aspect of the present invention provides a composition for diagnosing cancer including a preparation for measuring the level of an MSH2 or MSH6 gene, its protein, or a peptide constituting the protein.

Yet another aspect of the present invention provides a method for screening a substance for prevention or treatment of cancer including (a) contacting a sample to be analyzed with cells including an MSH2 gene or its protein; or an MSH6 gene or its protein; (b) measuring the expression level of the MSH2 or MSH6 gene, the protein expression level, or the protein activity; and (c) determining the sample as a substance for preventing or treating cancer when the expression level of the MSH2 or MSH6 gene, the protein expression level, or the protein activity is reduced as a result of the measurement in step (b).

### [Advantageous Effects]

According to the present invention, an MSH2 or MSH6 inhibitor is confirmed to: inhibit tumor cell growth and proliferation in various carcinoma cells, including hepatocellular carcinoma, and in particular, inhibit migration, invasion, and tumorigenic activity in hepatocellular carcinoma; and increase DNMT1 expression and reduce KLF4 expression, when MSH2 or MSH6 expression was increased, and thus can be used as a pharmaceutical composition for the prevention and treatment of cancer.

### [Description of Drawings]

FIG. 1 is a diagram briefly illustrating a process of identifying carcinomas with high expression of MSH2 or MSH6 among 33 types of carcinomas of TCGA according to an embodiment of the present invention.
FIG. 2 is a diagram illustrating evaluation results of cytotoxicity according to MSH2 inhibition in carcinomas with high expression of MSH2 and MSH6 according to an embodiment of the present invention.
FIG. 3 is a diagram illustrating evaluation results of cell proliferation ability according to MSH2 inhibition in carcinomas with high expression of MSH2 and MSH6 according to an embodiment of the present invention.
FIG. 4 is a diagram illustrating evaluation results of cell viability according to MSH2 inhibition in carcinomas with high expression of MSH2 and MSH6 according to an embodiment of the present invention.
FIG. 5 is a diagram illustrating comparative analysis results of MSH2 expression levels in liver cancer compared to a normal according to an embodiment of the present invention.
FIG. 6 is a diagram illustrating analysis results of growth, death, proliferation, migration, and invasion of tumor cells according to MSH2 inhibition in liver cancer cell lines with increased expression of MSH2 according to an embodiment of the present invention.
FIG. 7 is a diagram illustrating analysis results of cell cycle changes according to MSH2 inhibition in liver cancer cell lines with increased expression of MSH2 according to an embodiment of the present invention.
FIG. 8 is a diagram illustrating evaluation results of cytotoxicity and cell proliferation ability according to MSH2 overexpression in normal cells and liver cancer cell lines with decreased expression of MSH2 according to an embodiment of the present invention.
FIG. 9 is a diagram illustrating analysis results of a correlation between MSH2 and DNMT1 according to an embodiment of the present invention.
FIG. 10 is a diagram illustrating comparative analysis results of MSH6 expression levels in liver cancer compared to a normal according to an embodiment of the present invention.
FIG. 11 is a diagram illustrating analysis results of growth, death, proliferation, migration, and invasion of tumor cells according to MSH6 inhibition in liver cancer cell lines with increased expression of MSH6 according to an embodiment of the present invention.
FIG. 12 is a diagram illustrating analysis results of cell cycle changes according to MSH6 inhibition in liver cancer cell lines with increased expression of MSH6 according to an embodiment of the present invention.
FIG. 13 is a diagram illustrating evaluation results of cytotoxicity and cell proliferation ability according to MSH6 overexpression in normal cells and liver cancer cell lines with decreased MSH6 expression according to an embodiment of the present invention.
FIG. 14 is a diagram illustrating analysis results of a correlation between MSH6 and DNMT1 according to an embodiment of the present invention.
FIG. 15 is a diagram illustrating analysis results of a correlation between DNMT1 and KLF4 according to an embodiment of the present invention.
FIG. 16 is a diagram illustrating analysis results of a correlation between HDAC6 and MSH2 according to an embodiment of the present invention.
FIG. 17 is a diagram illustrating analysis results of a correlation between HDAC6 and MSH6 according to an embodiment of the present invention.
FIG. 18 is a diagram illustrating analysis results of a correlation between HDAC2 or KLF6 and HDAC6 according to an embodiment of the present invention.
FIG. 19 is a diagram illustrating analysis results of competitive binding of HDAC2 or KLF6 to an HDAC6 promoter.
FIG. 20 is a diagram confirming *in vivo* that an HDAC6-MSH2/MSH6-DNMT1-KLF4 axis regulates HCC tumorigenesis according to an embodiment of the present invention.
FIG. 21 is a diagram confirming in a mouse xenograft model that an HDAC6-MSH2/MSH6-DNMT1-KLF4 axis regulates HCC tumorigenesis according to an embodiment of the present invention.

### [Best Mode of the Invention]

Hereinafter, embodiments of the present invention will be described in detail with reference to the accompanying drawings. However, the following embodiments are presented as examples for the present invention, and when it is determined that a detailed description of well-known technologies or configurations known to those skilled in the art may unnecessarily obscure the gist of the present invention, the detailed description thereof may be omitted, and the present invention is not limited thereto. Various modifications and applications of the present invention are possible within the description of claims to be described below and the equivalent scope interpreted therefrom.

Terminologies used herein are terminologies used to properly express preferred embodiments of the present invention, which may vary according to a user, an operator's intention, or customs in the art to which the present invention pertains. Therefore, these terminologies used herein will be defined based on the contents throughout the specification. Throughout the specification, unless explicitly described to the contrary, when a certain part "comprises" a certain component, it will be understood to imply the inclusion of stated elements but not the exclusion of any other elements.

The present invention provides a pharmaceutical composition for preventing or treating cancer including as an active ingredient an inhibitor for an MutS homolog2 (MSH2) gene or protein; or an inhibitor for an MutS homolog6 (MSH6) gene or protein.

In addition, the MSH2 gene may be present on human chromosome 2 and may be located on preferably 47,403,067 to 47,634,501 of human chromosome 2 (Genome Reference Consortium: GRCh38.p13, NCBI Reference Sequence: NC_000002.12, Gene ID: 4436).

In addition, the MSH6 gene may be present on human chromosome 2 and may be located on preferably 47,783,145 to 47,806,954 of human chromosome 2 (Genome Reference Consortium: GRCh38.p13, NCBI Reference Sequence: NC_000002.12, Gene ID: 2956).

As used herein, the term `MSH2 (MutS homolog2)' is a DNA mismatch repair protein and forms a hetero dimer with MSH2 (MutS homolog6) [81]and is one of proteins that form a complex involved in the repair of missense base pairs of DNA.

As used herein, the term `MSH6 (MutS homolog6)' is a DNA mismatch repair protein and forms a hetero dimer with MSH2 (MutS homolog2) and is one of proteins that form a complex involved in the repair of missense base pairs of DNA.

In addition, the inhibitor may be selected from the group consisting of siRNA, shRNA, miRNA, antisense oligonucleotide, aptamers, antibodies, and combinations thereof, which are specific for MSH2 or MSH6, but is not limited thereto.

In addition, the MSH2-specific siRNA may be represented by a base sequence set forth in SEQ ID NO: 1 but is not limited thereto.

In addition, the MSH6-specific siRNA may be represented by a base sequence set forth in SEQ ID NO: 2 but is not limited thereto.

In addition, the composition may inhibit the expression of DNMT1 or increase the expression of KLF4.

As used herein, the term `DNA methyltransferase (DNMT)' is an enzyme that serves to bind to DNA by transferring a methyl group (-CH₃). Abnormal DNA hypermethylation is known to be a common phenomenon that occurs in most cancer cells, and accordingly, the activity of DNMT may be inhibited to suppress hypermethylation of cancer suppressor genes, and the cancer suppressor genes are normally re-expressed to suppress the proliferation of cancer cells.

As used herein, the term 'DNA (cytosine-5)-methyltransferase 1 (DNMT1)' is a major enzyme that maintains methylation patterns after DNA replication and serves to transfer a methyl group to a cytosine nucleotide in genomic DNA. In the present invention, the DNMT1 serves to suppress the expression of KLF4 by inducing methylation of a KLF4 promoter in cancer cells.

In an embodiment, the DNMT1 may interact with DNMT3A and DNMT3B of the DNMT family.

As used herein, the term `Kruppel-like factor 4 (KLF4)' may activate transcription by interacting with specific transcription factors and is well-known as a tumor suppressor.

In an embodiment, the KLF4 may interact with MSH2 and DNMT1.

As used herein, the term "expression inhibition" means causing a decrease in expression (to mRNA) or translation (to protein) of a target gene, and preferably, means that the expression of the target gene becomes undetectable or exists at an insignificant level thereby.

In addition, the composition may inhibit the growth, proliferation, migration, and invasion of tumor cells, but is not limited thereto.

In addition, the cancer may be selected from the group consisting of liver cancer, lung cancer, head and neck cancer, colon cancer, sarcoma, stomach cancer, and gallbladder cancer, preferably liver cancer, more preferably hepatocellular carcinoma, but is not limited thereto.

In addition, the cancer may be liver cancer of a patient with decreased expression of Kruppel-like factor 6 (KLF6), Histone deacetylase 6 (HDAC6), or Kruppel-like factor 4 (KLF4), or increased expression of Histone deacetylase 2 (HDAC2), mutS homolog2 (MSH2), or DNA (cytosine-5)-Methyltransferase 1 (DNMT1). The liver cancer may be hepatocellular carcinoma, preferably hepatocellular carcinoma of a stage I, II, III, IVA or IVB phase, and more preferably hepatocellular carcinoma of stage III and IV phases which are more difficult to be treated than an early stage but is not limited thereto.

As used herein, the term "cancer" refers to a condition in which a problem occurs in the regulatory function of the cell itself to cause excessive proliferation of abnormal cells that should normally die, and the abnormal cells are invaded into surrounding tissues and organs to form lumps and destroy or deform existing structures and is used in the same sense as malignant tumor.

As used herein, the term "prevention" refers to all actions that inhibit or delay the occurrence, development, and recurrence of cancer by administration of the composition according to the present invention.

As used herein, the term "treatment" means all actions that improve or beneficially change the symptoms of the cancer and its complications by administration of the composition according to the present invention. Those skilled in the art to which the present invention pertains will be able to determine the degree of improvement, enhancement, and treatment by knowing the exact criteria of a disease for which the composition of the present invention is effective by referring to data presented by the Korean Academy of Medical Sciences, etc.

The composition according to the present invention may include a pharmaceutically effective dose of MSH2 inhibitor or MSH6 inhibitor alone or may include one or more pharmaceutically acceptable carriers, excipients, or diluents. The pharmaceutically effective dose refers to an amount enough to prevent, improve, and treat symptoms of immune diseases. The "pharmaceutically acceptable" refers to a composition that is physiologically acceptable and does not normally cause an allergic reaction, such as gastrointestinal disorder, dizziness, etc., or a similar reaction thereto when administered to humans.

In addition, the composition including the pharmaceutically acceptable carrier may be various oral or parenteral formulations. When the composition is formulated, the formulations may be prepared by using diluents or excipients, such as a filler, an extender, a binder, a wetting agent, a disintegrating agent, a surfactant, etc., which are generally used. The carriers, excipients and diluents may be at least one selected from the group consisting of lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinyl pyrrolidone, physiological saline, methylhydroxybenzoate, propylhydroxybenzoate, talc, magnesium stearate and mineral oil, dextrin, calcium carbonate, propylene glycol, and liquid paraffin, but are not limited thereto, and may be used with all conventional carriers, excipients or diluents. The ingredients may be added independently or in combination with the MSH2 inhibitor as the active ingredient.

Solid formulations for oral administration may include tablets, pills, powders, granules, capsules, and the like, and the solid formulations may be prepared by mixing at least one compound with at least one excipient, for example, starch, calcium carbonate, sucrose or lactose, gelatin, and the like. Further, lubricants such as magnesium stearate and talc may also be used in addition to simple excipients. Liquid formulations for oral administration may correspond to a suspension, an oral liquid, an emulsion, a syrup, and the like, and may include various excipients, for example, a wetting agent, a sweetener, an aromatic agent, a preserving agent, and the like, in addition to water and liquid paraffin which are commonly used as simple diluents.

Formulations for parenteral administration include a sterile aqueous solution, a non-aqueous solvent, a suspension, an emulsion, a lyophilizing agent, a suppository, and the like. As the non-aqueous solvent and the suspension, propylene glycol, polyethylene glycol, vegetable oil such as olive oil, injectable ester such as ethyl oleate, and the like may be used. As a base of the suppository, witepsol, macrogol, Tween 61, cacao butter, laurinum, glycerol, gelatin, and the like may be used.

Further, the pharmaceutical composition of the present invention may have any one formulation selected from the group consisting of tablets, pills, powders, granules, capsules, suspensions, oral liquids, emulsions, syrups, sterilized aqueous solutions, non-aqueous solvents, suspensions, emulsions, lyophilized agents, and suppositories. As a base of the suppository, witepsol, macrogol, Tween 61, cacao butter, laurinum, glycerol, gelatin, and the like may be used.

The pharmaceutical composition of the present invention may be administered orally or parenterally (e.g., intravenously, subcutaneously, intraperitoneally, or topically) according to a desired method, and the range of the dose varies depending on the body weight, age, sex, and health condition of a patient, a diet, an administration time, an administration method, an excretion rate, the severity of a disease, and the like.

The pharmaceutical composition of the present invention may be used alone or in combination with surgery, hormone therapy, chemotherapy, and methods of using biological response modifiers for a prevention or treatment effect of cancer.

Furthermore, the present invention provides a method for treating liver cancer including administering the pharmaceutical composition to a subject.

In the present invention, the "subject" is not particularly limited as long as the subject is any object for the purpose of preventing or treating cancer, and includes animals including humans, for example, mammals including non-primates (e.g., cows, pigs, horses, cats, dogs, rats and mice) and primates (e.g., monkeys such as cynomolgous monkeys and chimpanzees).

The pharmaceutical composition of the present invention may be administered in a therapeutically effective dose or a pharmaceutically effective dose.

As used herein, the term "therapeutically effective dose" means an amount of a pharmaceutically acceptable salt of the composition effective for preventing or treating a target disease, and the therapeutically effective dose of the composition of the present invention may vary depending on many factors, such as a method of administration, a target site, a condition of a patient, and the like. Accordingly, when used in the human body, the dose should be determined as an appropriate amount in consideration of both safety and efficiency. It is also possible to estimate the amount used in humans from the effective dose determined through animal experiments. These matters to be considered when determining the effective dose are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The term 'pharmaceutically effective dose' used herein refers to an amount enough to treat a disease at a reasonable benefit/risk ratio applicable to medical treatment and that does not cause side effects. The effective dose level may be determined according to factors including the health condition of a patient, the type and severity of a disease, the activity of a drug, the sensitivity to a drug, an administration method, an administration time, an administration route, an excretion rate, duration of treatment, and drugs used in combination or simultaneously, and other factors well-known in the medical field. The composition of the present invention may be administered as an individual therapeutic agent or in combination with other therapeutic agents and may be administered sequentially or simultaneously with conventional therapeutic agents and may be administered singly or multiply. It is important to administer an amount capable of obtaining a maximum effect with a minimal amount without side effects by considering all the factors, which may be easily determined by those skilled in the art.

Further, the present invention provides a composition for diagnosing cancer including a preparation for measuring the level of an MSH2 or MSH6 gene, its protein, or a peptide constituting the protein.

In addition, the cancer may be selected from the group consisting of liver cancer, lung cancer, head and neck cancer, colon cancer, sarcoma, stomach cancer, and gallbladder cancer, preferably liver cancer, more preferably hepatocellular carcinoma, but is not limited thereto.

As used herein, the term "diagnosis" includes determining the susceptibility of a subject to a specific disease or disorder, determining whether a subject currently has a specific disease or disorder, determining the prognosis of a subject suffering from a specific disease or disorder (e.g., identifying a pre-metastatic or metastatic cancer condition, determining a stage of cancer, or determining the responsiveness of cancer to be treated), or therametrics (e.g., monitoring the condition of a subject to provide information about therapeutic efficacy).

In addition, the preparation for measuring the gene level may be a primer or probe that specifically binds to the gene but is not limited thereto.

Further, the measurement of the gene level may be performed by at least one method selected from the group consisting of reverse transcription polymerase chain reaction (RT-PCR), competitive polymerase chain reaction, real-time polymerase chain reaction, nuclease protection assay (RNase, S1 nuclease assay), in situ hybridization method, DNA microarray using method, Northern blot, Western blot, Enzyme Linked Immuno Sorbent Assay (ELISA), radioimmunoassay, immunodiffusion assay, immunoelectrophoresis, tissue immunostaining, immunoprecipitation assay, complement fixation assay, FACS, mass spectrometry, and protein microarray using method, but is not limited thereto.

The MSH2 protein may be constituted by all or part of peptides consisting of amino acids translated into a nucleic acid sequence located on 47,403,067 to47,634,501 of human chromosome 2, and includes functional equivalents and variants of the MSH2 protein. In addition, the peptides constituting the MSH2 protein refer to fragments of the MSH2 protein and include any peptide constituting the MSH2 protein without limitation.

The MSH6 protein may be constituted by all or part of peptides consisting of amino acids translated into a nucleic acid sequence located on 47,783,145 to47,806,954 of human chromosome 2, and includes functional equivalents and variants of the MSH6 protein. In addition, the peptides constituting the MSH6 protein refer to fragments of the MSH6 protein and include any peptide constituting the MSH6 protein without limitation.

The functional equivalents of the protein include proteins and polypeptides that do not change the activity of protein molecules as a whole, and proteins that may perform the same functional action are included within the scope of the present invention. The variant refers to a protein having a different sequence from an MSH2 native amino acid sequence by deletion, insertion, non-conservative or conservative substitution, or a combination thereof of at least one amino acid residue. For example, the variant is one in which one or several amino acids are deleted, substituted, or added in the above amino acid sequence, and that has at least 95%, preferably at least 98%, and more preferably at least 99% identity with the amino acid sequence, etc. Here, the "identity" refers to a ratio (%) of the number of identical amino acid residues of the other amino acid sequence to the total number of amino acid residues of one amino acid sequence, including the number of gaps, when the two amino acid sequences are aligned to achieve the highest degree of identity with or without introducing gaps. In addition, "several" refers to an integer of 2 to 10, for example, 2 to 7, 2 to 5, 2 to 4, or 2 to 3. Specific examples of natural variants may include variants based on polymorphisms such as single nucleotide polymorphism (SNP), splice variants, and the like. It is preferable that the substitution is a conservative amino acid substitution. This is because the conservative amino acid substitution may have a structure or property substantially equivalent to MSH2 or MSH6 having the amino acid sequence. The conservative amino acids are known as non-polar amino acids (glycine, alanine, phenylalanine, valine, leucine, isoleucine, methionine, proline, tryptophan), polar amino acids (amino acids other than non-polar amino acids), charged amino acids (acidic amino acids (aspartic acid, glutamic acid), and basic amino acids (arginine, histidine, lysine)) and uncharged amino acids (amino acids other than charged amino acids), aromatic amino acids (phenylalanine, tryptophan, tyrosine), branched amino acids (leucine, isoleucine, valine), aliphatic amino acids (glycine, alanine, leucine, isoleucine, valine), etc.

In addition, the proteins may include proteins with increased structural stability against heat, pH, etc. of the protein or increased protein activity by mutations or modifications in the amino acid sequence.

In addition, the preparation for measuring the level of the protein or peptide constituting the protein may be any one selected from the group consisting of antibodies, antisense RNA, aptamers, and compounds specific for the protein or fragment peptide, but is not limited thereto.

As used herein, the term "antibody" refers to a specific protein molecule directed against an antigenic site. For the purposes of the present invention, the antibody refers to an antibody that specifically binds to the MSH2 protein or MSH6 protein, and includes all polyclonal antibodies, monoclonal antibodies, and recombinant antibodies.

Further, the antibody of the present invention includes a functional fragment of the antibody molecule as well as a complete form having two full-length light chains and two full-length heavy chains. The functional fragments of the antibody molecule refer to fragments having at least an antigen binding function, and may be Fab, F(ab'), F(ab')2, Fv, etc.

In addition, the polyclonal antibody may be produced by injecting the protein expressed by the gene or its fragment as an immunogen into an external host according to a method known to those skilled in the art. The external host includes mammals such as mice, rats, sheep, or rabbits. The immunogen is injected intramuscularly, intraperitoneally, or subcutaneously, and generally administered with an adjuvant to increase antigenicity. The serum is collected regularly from the external host, and the serum showing improved titer and specificity for the antigen is collected or antibodies are separated and purified therefrom.

In addition, the monoclonal antibody may be prepared by techniques for producing immortalized cell lines by fusion known to those skilled in the art. For example, mice are immunized with the protein expressed by the gene, or peptides are synthesized and bound to bovine serum albumin to immunize mice. The monoclonal antibodies may be prepared by producing immortalized hybridomas by fusing antigenproducing B lymphocytes isolated from mice with human or mouse myeloma, identifying whether to produce monoclonal antibodies using indirect enzyme-linked immunoabsorbent assays (ELISA) with hybridoma cells, and selecting and culturing positive clones and then isolating and purifying the antibody or injecting the antibody into the abdominal cavity of a rat and then collecting the ascites.

In addition, the measurement of the protein level may be performed by at least one selected from the group consisting of Western blot, enzyme linked immunosorbent assay (ELISA), radioimmunoassay (RIA), radioimmunodiffusion, Ouchterlony immunodiffusion, rocket immunoelectrophoresis, tissue immunostaining, immunoprecipitation assay, complement fixation assay, fluorescence activated cell sorter (FACS), and protein chips, but is not limited thereto.

Furthermore, the present invention provides an information providing method for predicting the diagnosis or prognosis of liver cancer including confirming the expression level of MSH2 or MSH6 in a biological sample; and comparing the expression level of MSH2 or MSH6 with that of a normal sample.

In addition, the method may further include determining that the risk of suffering from liver cancer is high when MSH2 or MSH6 is overexpressed by at least 1.5-fold greater than that of the normal sample.

In addition, the biological sample may include samples such as tissues, cells, whole blood, serum, plasma, saliva, sputum, cerebrospinal fluid, urine, etc.

Finally, the present invention provides a method for screening a substance for prevention or treatment of cancer including (a) contacting a sample to be analyzed with cells including an MSH2 gene or its protein; or an MSH6 gene or its protein; (b) measuring the expression level of the MSH2 or MSH6 gene, the protein expression level, or the protein activity; and (c) determining the sample as a substance for preventing or treating cancer when the expression level of the MSH2 or MSH6 gene, the protein expression level, or the protein activity is reduced as a result of the measurement in step (b).

In addition, the cancer may be selected from the group consisting of liver cancer, lung cancer, head and neck cancer, colon cancer, sarcoma, stomach cancer, and gallbladder cancer, but is not limited thereto.

As used herein, the term "candidate substance" refers to an unknown substance used for screening, in order to examine whether to affect the expression of the MSH2 or MSH6 gene; or the activity of the MSH2 or MSH6 protein. The candidate substance is not limited thereto, but may include chemicals, peptides, and natural extracts. The candidate substance analyzed by the screening method of the present invention may be a single compound or a mixture of compounds and may be obtained from a library of synthetic or natural compounds.

Further, step (b) may be detecting or measuring at least one selected from the group consisting of an MSH2 or MSH6 gene expression level, an MSH2 or MSH6 protein expression level, other proteins affected by the MSH2 or MSH6 protein, interaction of the MSH2 or MSH6 protein with other proteins, a complex of the MSH2 or MSH6 protein and other proteins, and a complex of other proteins affected by the MSH2 or MSH6 protein, but is not limited thereto.

In addition, the expression level of the MSH2 or MSH6 gene or protein may be measured by confirming the expression level of mRNA or the level of the protein encoded by the gene. The amount of mRNA may be confirmed using primer pairs or probes, and an analysis method thereof includes RT-PCR, competitive RT-PCR, real-time RT-PCR, RNase protection assay (RPA), Northern blotting, DNA chips, etc., but is not limited thereto. The amount of protein encoded by the gene may be confirmed using an antibody that specifically binds to the protein. The analysis method thereof includes Western blot, enzyme linked immunosorbent assay (ELISA), radioimmunoassay (RIA), radioimmunodiffusion, Ouchterlony immunodiffusion method, rocket immunoelectrophoresis, tissue immunostaining, immunoprecipitation assay, complement fixation assay, FACS, protein chips, etc., but is not limited thereto.

In addition, step (c) is a step of selecting a candidate substance that decreases the expression or activity of the MSH2 or MSH6 protein among the candidate substances and may measure that the expression of the MSH2 or MSH6 gene or the activity of the MSH2 or MSH6 protein is downregulated by the candidate substance to determine the candidate substance as the substance for prevention or treatment of cancer.

The screening method of the present invention may be performed in various manners, and in particular, may be performed in a high throughput method according to various binding assays known in the art. In the screening method of the present invention, the candidate substance or MSH2 protein may be labeled with a detectable label. For example, the detectable label includes chemical labels (e.g. biotin), enzyme labels (e.g., horseradish peroxidase, alkaline phosphatase, peroxidase, luciferase, β-galactosidase and β-glucosidase), radioactive labels (e.g., C14, I125, P32 and S35), fluorescent labels (e.g., coumarin, fluorescein, fluorescein isothiocyanate (FITC), rhodamine 6G, rhodamine B, 6-carboxy-tetramethylrhodamine (TAMRA), Cy-3, Cy-5, Texas Red, Alexa Fluor, 4,6-diamidino-2-phenylindole (DAPI), HEX, TET, Dabsyl and FAM), luminescent labels, chemiluminescent labels, fluorescence resonance energy transfer (FRET) labels, or metallic labels (e.g., gold and silver). In the case of using the MSH2 protein or candidate substance labeled with the detectable label, whether binding occurs between the MSH2 or MSH6 protein and the candidate substance may be analyzed by detecting a signal from the label. For example, when alkaline phosphatase is used as a label, the signal is detected using a chromogenic substrate such as bromochloroindolyl phosphate (BCIP), nitro blue tetrazolium (NBT), naphthol-AS-B1-phosphate and enhanced chemifluorescence (ECF). When horse radish peroxidase is used as the label, the signal is detected using a substrate such as chloronaphthol, aminoethylcarbazole, diaminobenzidine, D-luciferin, bis-N-methylacridinium nitrate (lucigenin), resorufin benzyl ether, luminol, Amplex Red reagent (10-acetyl-3,7-dihydroxyphenoxazine), p-phenylenediamine-HCl and pyrocatechol (YR), tetramethylbenzidine (TMB), 2,2-Azine-di[3-ethylbenzthiazoline sulfonate] (ABTS), o-phenylenediamine (OPD) and naphthol/pyronine. Alternatively, the binding of the candidate to the MSH2 protein may also be analyzed without labeling interactants. For example, a microphysiometer may be used to analyze whether the candidate substance binds to the MSH2 protein. The microphysiometer is an analytical tool that measures the rate at which cells acidify the environment using a light-addressable potentiometric sensor (LAPS). Changes in acidification rate may be used as an indicator of binding between the candidate substance and the MSH2 or MSH6 protein (McConnell et al., Science 257:19061912 (1992)).

### [Modes of the Invention]

Hereinafter, the present invention will be described in more detail through Examples. However, these Examples are more specifically illustrative of the present invention, and the scope of the present invention is not limited to these Examples.

### Example 1. Confirmation of expression of cancer suppressor gene MSH2 or MSH6 in large cohort RNA seq data

### 1-1. Confirmation of expression of cancer suppressor gene MSH2 or MSH6 in large cohort RNA seq data

Among 33 carcinomas in RNA sequencing data available from The Cancer Genome Atlas (TCGA), carcinomas in which the expression of MSH2 and MSH6 increased at least 1.5-fold in cancer patients compared to normal patients and carcinomas with clinical relevance were identified.

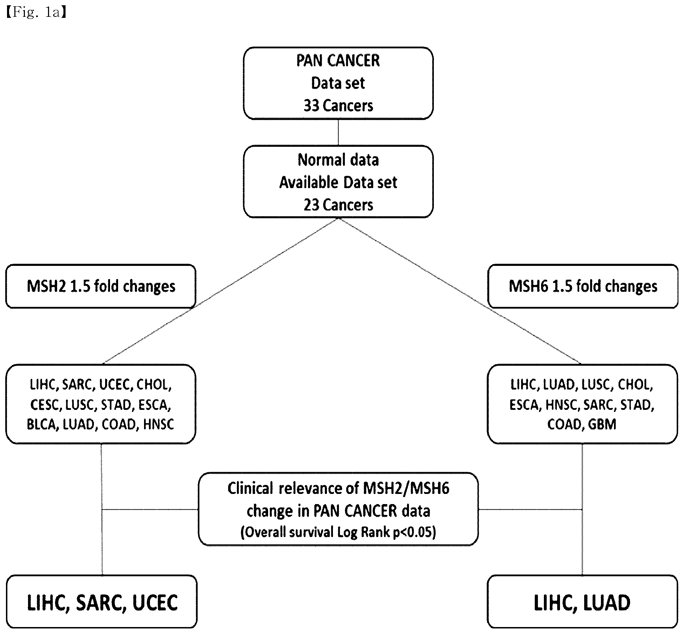

As a result, the expression level of MSH2 increased 1.5-fold in cancer patients compared to normal patients in 12 carcinomas LIHC, SARC, UCEC, CHOL, CESC, LUSC, STAD, ESCA, BLCA, LUAD, COAD, and HNSC, and it was shown that among the carcinomas, LIHC, SARC, and UCEC were overexpressed to affect the survival (FIG. 1A and Table 1).

The expression level of MSH6 increased 1.5-fold in cancer patients compared to normal patients in 10 carcinomas LIHC, LUAD, LUSC, CHOL, ESCA, HNSC, SARC, STAD, COAD, and GBM, and it was shown that among the carcinomas, LIHC and LUAD were overexpressed to affect the survival (FIG. 1A and Table 2).

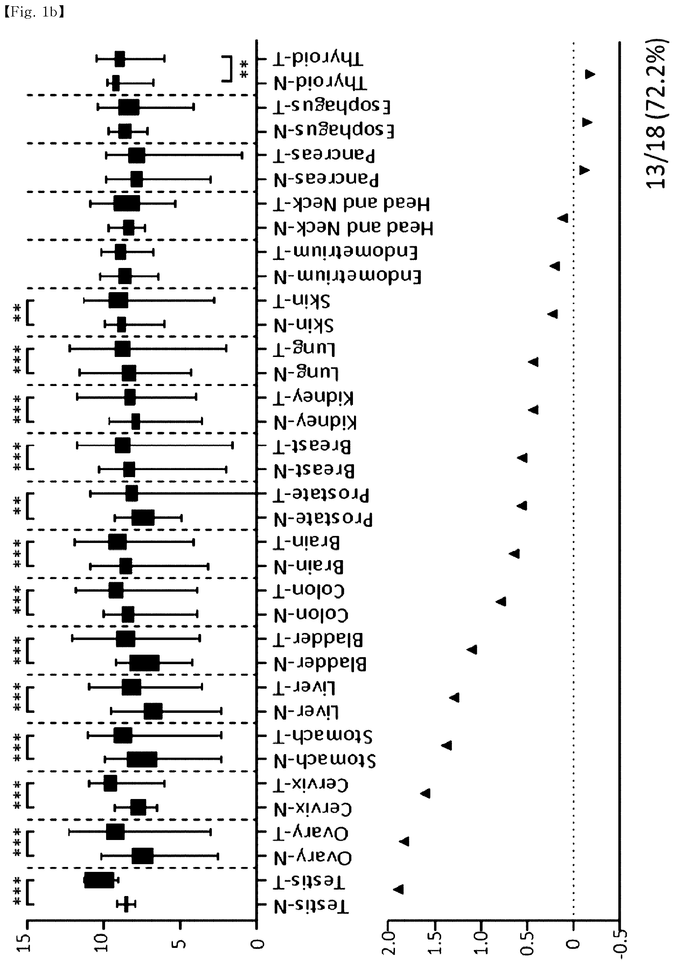

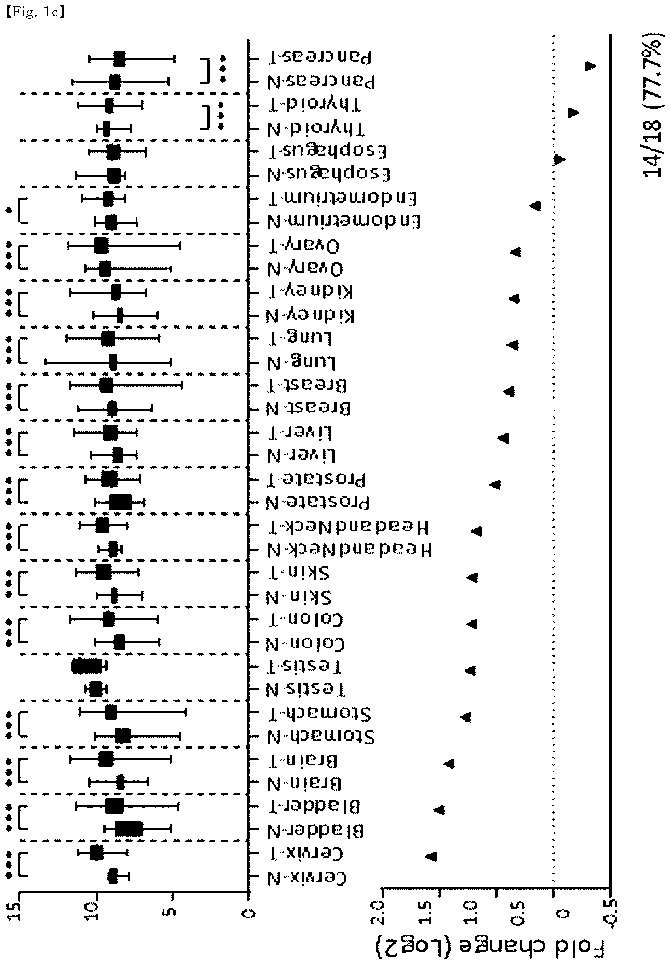

In addition, as a result of array data analysis using GENT2, it was confirmed that the expression of MSH2 was increased in 13 carcinomas, and the expression of MSH6 was increased in 14 carcinomas (FIGS. 1B and 1C).

**[Table 1]**

| Study Abbreviation | Study Name | MSH2 fold change | Overall survival Log Rank p < 0.05 |
|---|---|---|---|
| LIHC | Liver hepatocellular carcinoma | 2.264 | O |
| SARC | Sarcoma | 1.733 | O |
| UCEC | Uterine Corpus Endometrial Carcinoma | 1.616 | O |
| CHOL | Cholangiocarcinoma | 3.402 | |
| CESC | Cervical squamous cell carcinoma and endocervical adenocarcinoma | 3.104 | |
| LUSC | Lung squamous cell carcinoma | 2.253 | |
| STAD | Stomach adenocarcinoma | 2.17 | |
| ESCA | Esophageal carcinoma | 2.138 | |
| BLCA | Bladder Urothelial Carcinoma | 1.87 | |
| LUAD | Lung adenocarcinoma | 1.761 | |
| COAD | Colon adenocarcinoma | 1.637 | |
| HNSC | Head and Neck squamous cell carcinoma | 1.508 | |

**[Table 2]**

| Study Abbreviation | Study Name | MSH6 fold change | Overall survival Log Rank p < 0.05 |
|---|---|---|---|
| LIHC | Liver hepatocellular carcinoma | 1.633 | O |
| LUAD | Lung adenocarcinoma | 1.544 | O |
| LUSC | Lung squamous cell carcinoma | 2.39 | |
| CHOL | Cholangiocarcinoma | 1.984 | |
| ESCA | Esophageal carcinoma | 1.778 | |
| HNSC | Head and Neck squamous cell carcinoma | 1.749 | |
| SARC | Sarcoma | 1.701 | |
| STAD | Stomach adenocarcinoma | 1.57 | |
| COAD | Colon adenocarcinoma | 1.543 | |
| GBM | Glioblastoma multiforme | 1.502 | |

### Example 2. Confirmation of characteristics of MSH2 and MSH6 on tumorigenesis in various carcinomas

To confirm *in vitro* tumorigenesis in various carcinomas, MTT assay, BrdU assay and cell viability assay were performed for LIHC, LUAD, LUSC, CHOL, ESCA, HNSC, SARC, STAD, and COAD among carcinomas with high expression of MSH2 and MSH6 using si-MSH2 (SEQ ID NO: 1) and si-MSH6 (SEQ ID NO: 2).

Specifically, in order to transform various cancer cell lines with si-MSH2 or si-MSH6, the cancer cell lines were seeded in a 12-well plate at 40% confluency and then transformed with si-MSH2 or si-MSH6 to knockdown MSH2 or MSH6.

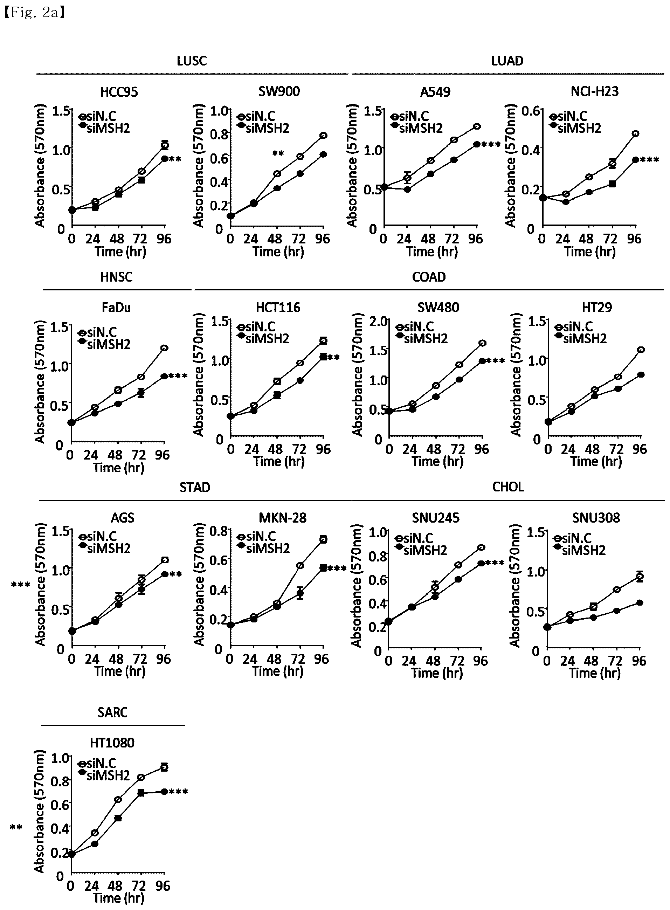

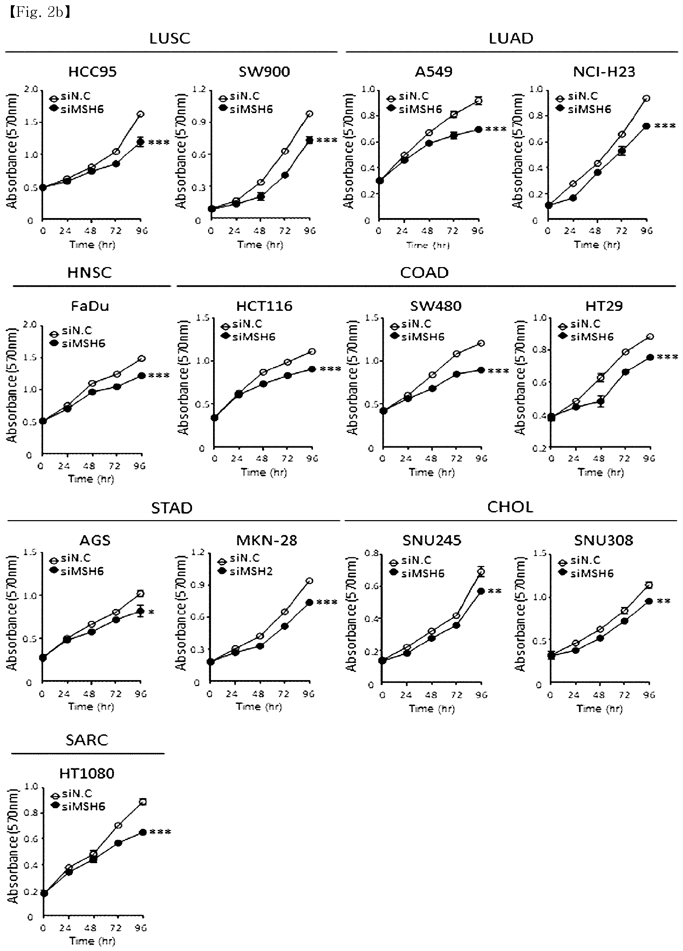

For MTT assay, the cells were dispensed in a 12-well plate, transfected with si-MSH2 or si-MSH6, and incubated for 1 hour with 0.5 mg/ml of an MTT [3-(4,5-dimethylthiazol-2-yl)-2,5-diphenyltetrazolium bromide] solution (Sigma) at 37°C in 5% CO₂ for 1 hour, and then absorbance was measured using a VICTOR3 Multilabel plate reader (PerkinElmer, Waltham, MA). As a result, it was confirmed that the growth was inhibited when MSH2 or MSH6 was knocked down (FIGS. 2A and 2B).

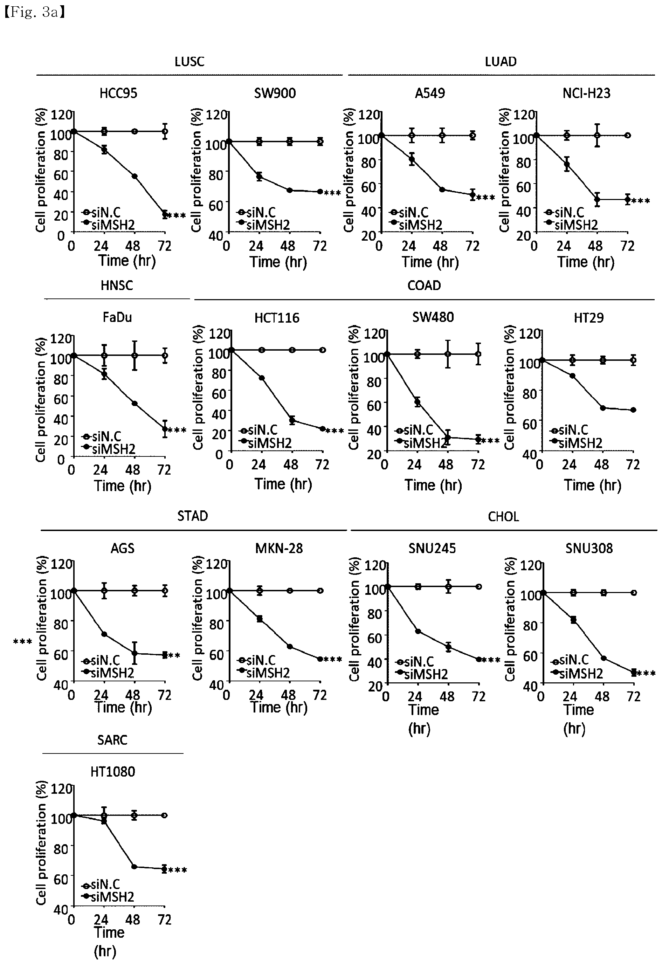

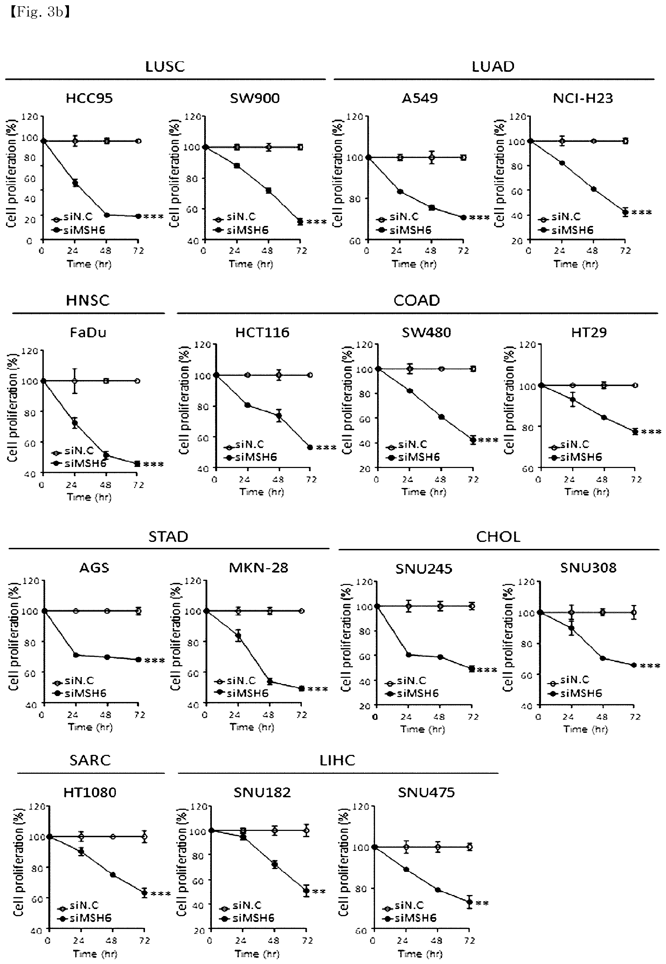

In addition, for the BrdU assay, various cancer cell lines transformed with si-MSH2 or si-MSH6 were treated with a 5-bromo-2'-deoxyuridine (BrdU) reagent for 6 hours and performed using a BrdU cell proliferation assay kit (Millipore) according to the manufacturer's protocol. As a result, it was confirmed that the proliferation was inhibited when MSH2 or MSH6 was knocked down (FIGS. 3A and 3B).

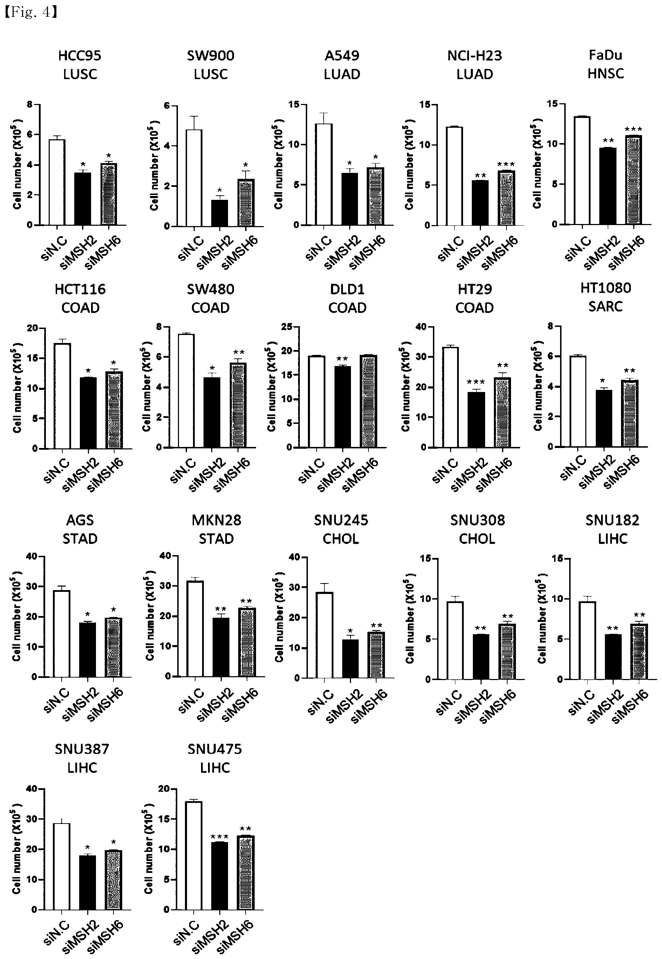

In addition, for the cell viability assay, various cells transformed with si-MSH2 or si-MSH6 were dispensed in a 6-well plate and cultured for 72 hours, and the cells were obtained with trypsin and stained with a trypan blue solution (Sigma, St. Louis, MO). After staining, the cells were counted with a hemocytometer (Marienfeld Superior, Lauda-Koonigshofen, Germany) to confirm cell survival. As a result, it was confirmed that the cell number was reduced when MSH2 or MSH6 was knocked down (FIG. 4). Here, in the case of the DLD1 cell line, the number of cells was not decreased, but it was not significant because the cell was an MSH2 null cell.

### Example 3. Verification of MSH2 with increased expression in liver cancer

### 3-1. Confirmation of MSH2 expression in HCC

To further analyze the expression of MSH2 in liver cancer, which ranked third in mortality among many carcinomas, TCGA, ICGC, GSE77314, and Catholic_mLIHC (GSE114564) data were analyzed.

To confirm the expression levels of subunit genes in hepatocellular carcinoma (HCC), data was obtained from a Gene Expression Omnibus (GEO) database (Accession Numbers: GSE77314 and GSE114564) of TCGA_LIHC (The Cancer Genome Atlas liver hepatocellular carcinoma project), ICGC_LIRI (International Cancer Genome Consortium liver CancerRIKEN, JP) and NCBI. Level 3 mRNA expression data of TCGA datasets HTSeq-FPKM was used to evaluate a gene expression level as a [log2 (fpkm + 1)] value.

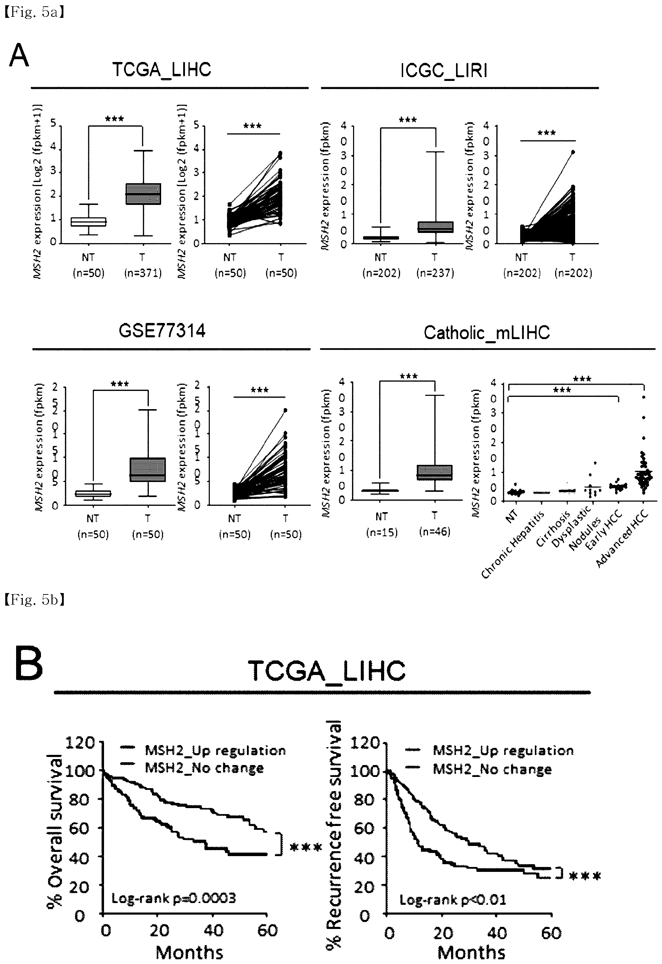

As a result, it was confirmed that the expression of MSH2 was increased in tumor compared to non-tumor, and the expression of MSH2 was also increased in paired samples of TCGA, ICGC, and GSE77314 data sets (FIG. 5A).

Subsequently, when survival analysis (Kaplan-Meier survival analysis) was performed on the TCGA data set, it was confirmed that patients with high expression of MSH2 had poor survival (FIG. 5B).

### 3-2. 40 matched-pair analysis

To identify the abnormal expression of MSH2 in HCC, total RNA was isolated from 40 matched-pairs (HCC tissue vs non-cancerous surrounding liver tissue) of HCC tissue and 13 HCC cell lines MIHA, L-02, Hep3B, Huh7, PLC/PRF/5, SNU182, SNU354, SNU368, SNU387, SNU398, SNU423, SNU449, and SNU475 using a TRIzol reagent (Invitrogen, Carlsbad, CA), and then cDNA was synthesized using a Tetro cDNA synthesis kit (Bioline, London, UK). qRT-PCR was performed with the synthesized cDNA. qRT-PCR was performed with a SensiFASTTM NoROX Kit (Bioline). In addition, total protein was extracted from the same HCC tissue and HCC cell line and the expression of MSH2 was also identified by performing Western blot.

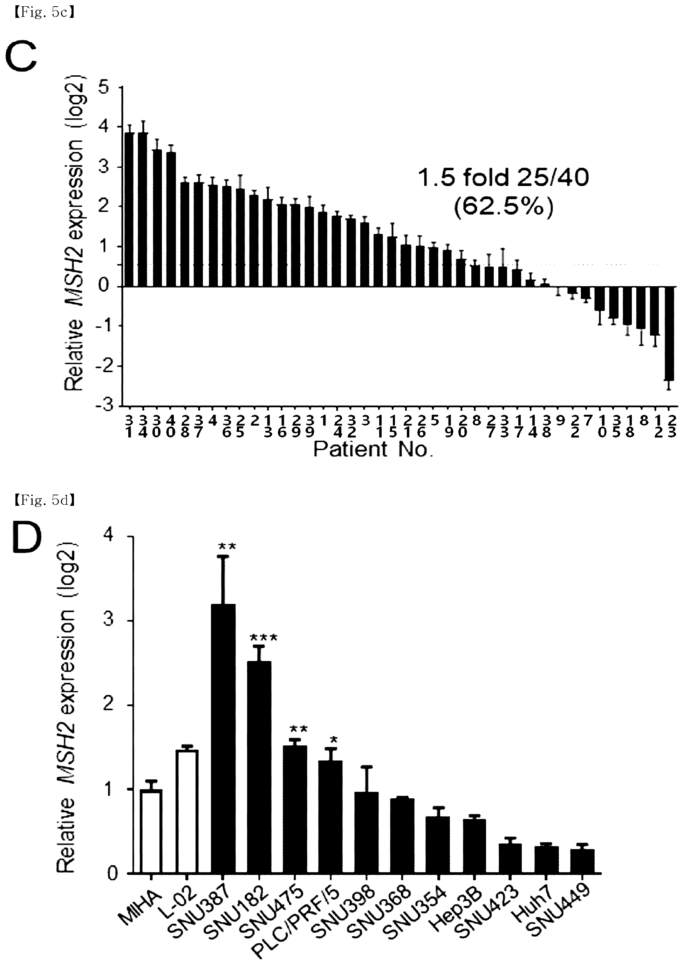

The 40 matched-pairs of HCC tissue and the non-cancerous surrounding liver tissue corresponding thereto were obtained from the National Biobank, received a written consent from each subject in accordance with the Declaration of Helsinki, and approved from the Institutional Review of Board (IRB) of Songeui Campus of Catholic Medical University (Approval number: MC19TESI0016). As a result, it was confirmed through qRT-PCR that 25 (62.5%) of 40 HCC patients showed significant overexpression of MSH2 compared to non-cancerous liver tissue (FIG. 5C).

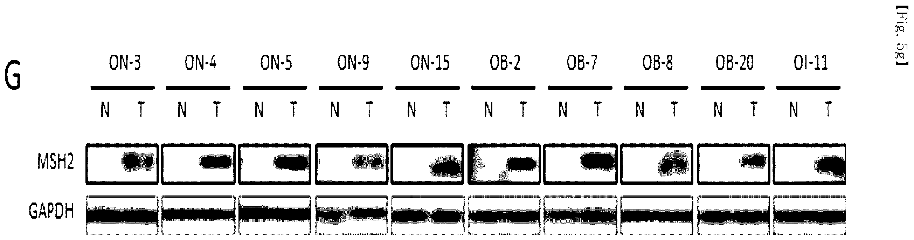

In addition, when proteins were extracted from normal liver tissue and liver cancer tissue of a liver cancer patient distributed from the National Biobank and the expression of MSH2 protein was confirmed through Western blot, it was confirmed that the expression of MSH2 protein was increased in tumor tissue (FIG. 5G).

### 3-3. Confirmation of MSH2 expression in liver cancer cell lines

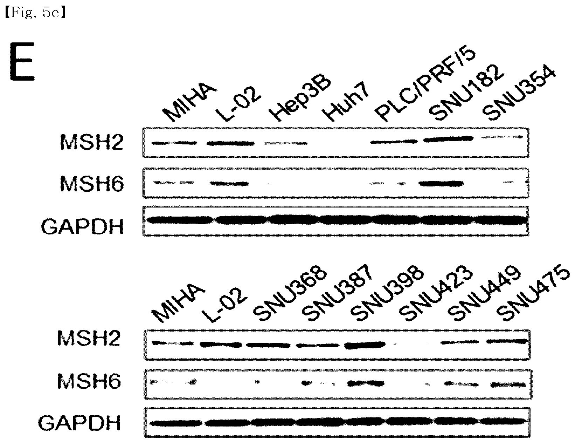

To confirm the MSH2 expression levels in various liver cancer cell lines, qRT-PCR and Western blot for MSH2 were performed. As a result, it was confirmed that the expression of MSH2 mRNA and protein was increased compared to normal hepatocytes (MIHA) (FIGS. 5D and 5E).

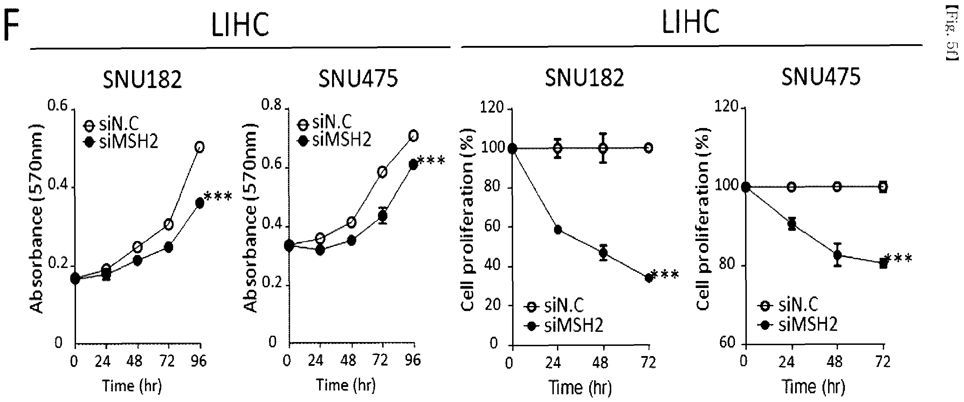

In addition, to confirm the effect of MSH2 inhibition in liver cancer with increased MSH2 expression, MSH2 was knocked down in liver cancer cell lines SNU182 and SNU475 with high MSH2 expression, and then MTT assay and BrdU assay were performed. As a result, it was confirmed that cell growth and cell proliferation decreased when MSH2 was inhibited in liver cancer cell lines with increased MSH2 (FIG. 5F).

### Example 4. Confirmation of characteristics of MSH2 in carcinogenic process

### 4-1. Confirmation of effect on tumor cell proliferation

To confirm the tumorigenic characteristics of MSH2 in liver cancer, clonogenic assay was performed on SNU182 and SNU475 transfected with si-MSH2.

For clonogenic analysis, a negative control group siRNA (si-Cont) and si-MSH2 transfected cells were seeded in a 6-well plate (1000 cells/well). After 12 days, the colonies were fixed with 1% paraformaldehyde for 30 minutes at room temperature and stained with 0.5% crystal violet for 1 hour at room temperature. The stained colonies were counted using commercial software (Clono counter).

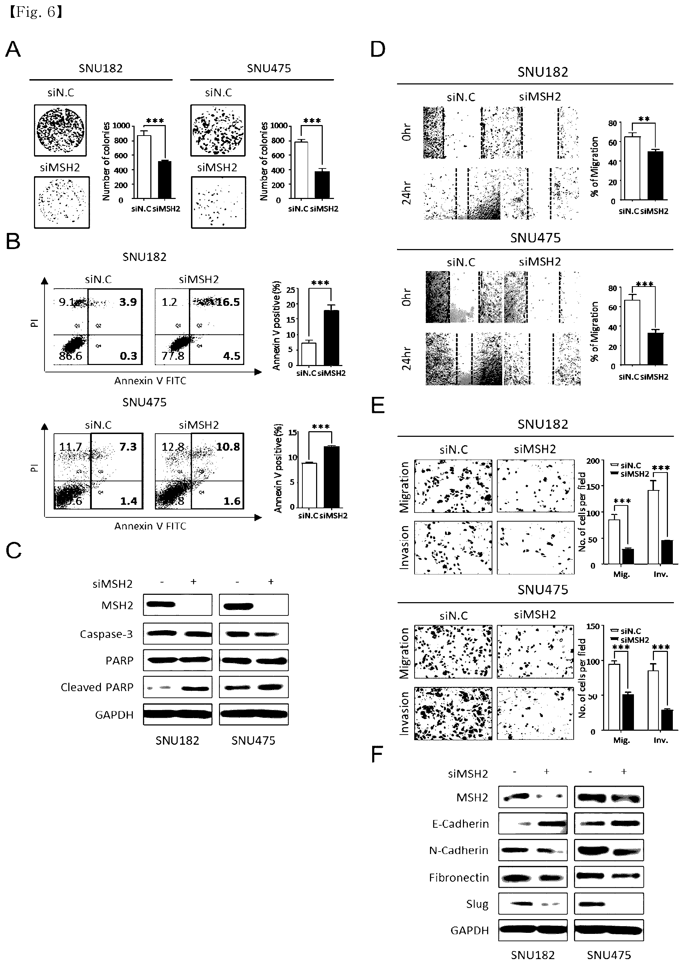

As a result, it was confirmed that colony formation was reduced when MSH2 was knocked down in liver cancer cell lines SNU182 and SNU475 with increased expression of MSH2 (FIG. 6A).

### 4-2. Confirmation of effect on apoptosis of tumor cells

Changes in apoptosis and cell cycle regulation caused by si-MSH2 transfection were confirmed. Apoptosis levels were measured using Annexin V-FITC Apoptosis Detection Kit I (BD Biosciences, San Jose, CA). Specifically, SNU182 and SNU475 cells were transfected with si-MSH2 and cultured for 48 hours, and then the cells were isolated with trypsin-EDTA and washed with PBS. The cells were resuspended in a 1x binding buffer and 100 µl of cells was transferred to a 5 ml culture tube to contain 1 × 10⁵ cells. Thereafter, 5 µl of Annexin V-FITC and 5 µl of PI (propidium iodide) solutions were added, and the cells were incubated at room temperature in dark conditions for 15 minutes. After incubation, 400 µl of the 1x binding buffer was added to each tube and the dead fraction was detected with a FACS Canto^{™} flow cytometer (BD Biosciences). In addition, the expression levels of apoptosis markers Caspase-3 and Cleaved PARP were confirmed in the SNU182 and SNU475 cells transfected with the si-MSH2.

As a result, it was confirmed through FACS analysis that apoptosis increased when MSH2 was knocked down (FIG. 6B), and similarly, as a protein expression analysis result, it was shown that PARP and caspase-3 decreased and cleaved PARP increased, and thus it was confirmed that PARP and caspase-3, which were involved in apoptosis, were activated (FIG. 6C).

### 4-3. Confirmation of effects on tumor cell migration and invasion

Since epithelial-to-mesenchymal transition (EMT) has been suggested as a key process in cancer progression along with cell growth, MSH2 was inhibited in HCC cells and a scratch wound healing assay was performed. Specifically, SNU182 and SNU475 cells were transfected with si-MSH2 and then incubated for 24 hours, and the cells were isolated with trypsin-EDTA and dispensed at 1 × 10⁶ per well in a 6-well plate. The next day, the cell monolayer was scraped with a sterilized micropipette tip. An initial scratch interval immediately after scratching and an interval 24 hours later were photographed using an IX71 photomicrograph (Olympus, Tokyo, Japan).

As a result, it was found that the wound healing effect was significantly reduced when MSH2 was knocked down in both SNU182 and SNU475 cells (FIG. 6D).

In addition, the migration and invasion characteristics of cancer cells were confirmed through modified Boyden chamber assay (BD Biosciences, San Jose, CA) and transwell invasion assay. The transwell invasion assay used a transwell plate and a cell culture insert (BD Bioscience). Specifically, Matrigel (BD Biosciences) was diluted with a coating buffer (0.01 M Tris, 0.7% NaCl, pH 8.0) to a concentration of 0.3 mg/ml, and 100 µl of Matrigel was coated on the cell culture insert. After incubation at 37°C for 1 hour, 1.5 × 10⁵ cells (motility assay) or 1.0 × 10⁵ cells (invasion assay) per well were seeded on the surface of the insert with a serum-free medium, and a lower transwell was seeded with 2% FBS as a chemoattractant. The plate was incubated at 37°C for 12 hours and then stained using a Diff-Quik staining kit (Sysmex, Kobe, Japan). Cell images were confirmed at X200 magnification using an Axiovert 200 inverted microscope (Zeiss, Oberkochen, Germany).

As a result, when MSH2 was knocked down in both SNU182 and SNU475 cells, it was found that the migration and invasion were significantly reduced (FIG. 6E).

To further verify the results, Western blot was performed on an EMT marker, and as a result, it was confirmed that N-Cadherin, Fibronectin, and Slug were decreased (FIG. 6F).

### 4-4. Confirmation of effect of changes in cell cycle regulation of tumor cells

To confirm changes in cell cycle regulation, cells were transfected with si-MSH2 and cultured for 48 hours, and then isolated with trypsin-EDTA. The isolated cells were washed with PBS fixed in 70% ethanol and resuspended in 200 µl of PBS containing 3 mg/ml of RNase A, 50 µg/ml of propidium iodide (PI, Sigma), and 1% Triton X-100. The cells were incubated at 37°C in dark conditions for 45 minutes. After incubation, the stained cell fraction was detected using a FACS CantoTM flow cytometer (BD Biosciences, San Jose, CA, USA).

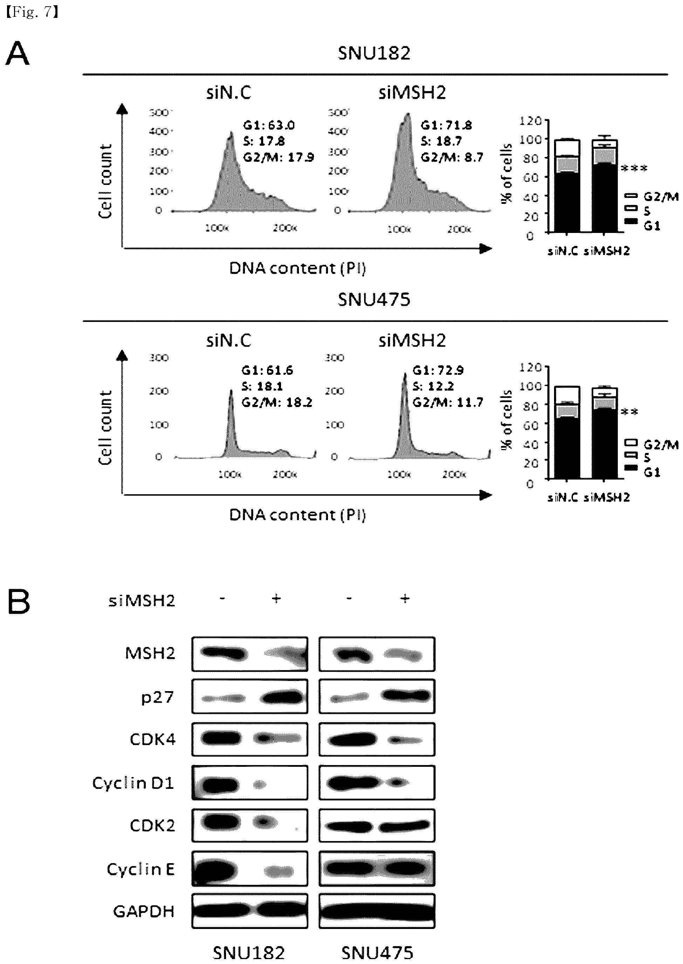

As a result, it was confirmed that a G1/S arrest was confirmed through FACS analysis when MSH2 was knocked down in liver cancer cell lines SNU182 and SNU475 with increased expression of MSH2 (FIG. 7A).

To further verify the results, Western blot was performed on cell cycle markers, and as a result, an increase in p27 and decreases in CDK4, Cyclin D1, CDK2, and Cyclin E were confirmed (FIG. 7B).

Through the results, it was found that abnormal expression of MSH2 contributed to the anti-apoptotic and metastatic characteristics of HCC.

### Example 5. Confirmation of oncogene characteristics of MSH2

To confirm the oncogenic function of MSH2, MSH2 was overexpressed through transient transfection in a normal liver cell line (MIHA) and a liver cancer cell line (Huh7) with decreased expression of MSH2, and then MTT assay and BrdU assay were performed.

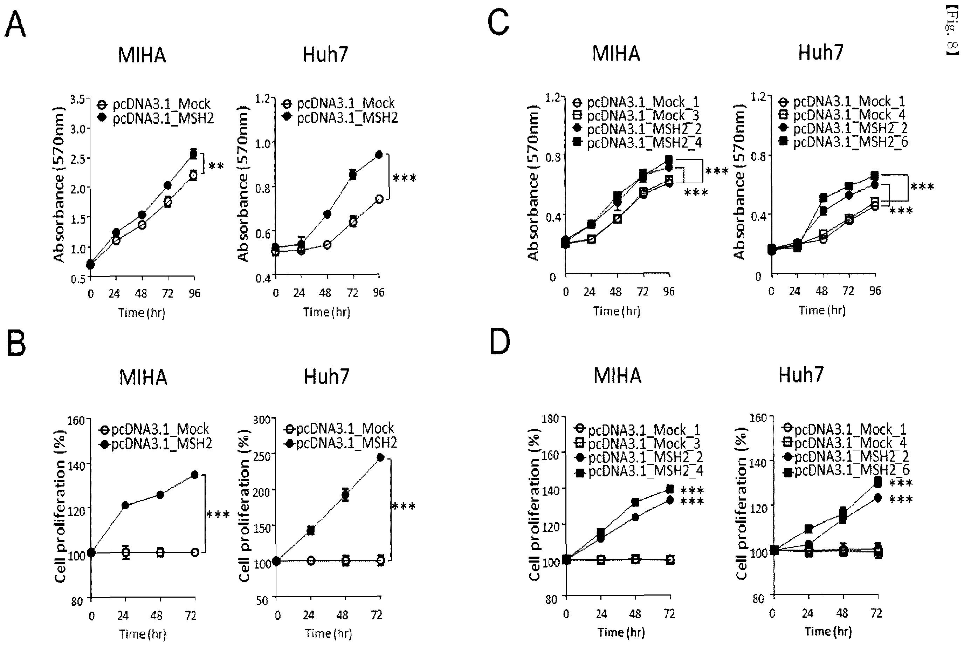

As a result, it was confirmed that cell growth and cell proliferation increased when MSH2 was overexpressed in the normal liver cell line and the liver cancer cell line with decreased MSH2 (FIG. 8A).

Next, a stable cell line was fabricated by overexpressing MSH2 continuously in the normal liver cell line (MIHA) and the liver cancer cell line (Huh7) with decreased expression of MSH2.

Specifically, a stable cell line was fabricated by transfecting an MSH2 plasmid, which was resistant to a drug 'Geneticin' to be selected, into the normal liver cell line (MIHA) and the liver cancer cell line (Huh7) with decreased expression of MSH2. When the Geneticin drug was treated after transfection, only the cells introduced with the plasmid survived to be selected, and only the surviving cells were subcultured, allowing only the cells introduced with the plasmid to be cultured. These selected cells continuously expressed MSH2.

As a result, similarly to the results, it was confirmed that cell growth and cell proliferation increased (FIG. 8B).

### Example 6. Search of target molecule of MSH2

### 6-1. Search of target molecule of MSH2

In Lynch syndrome, MSH2 was known to be methylated in a promoter region. To confirm the methylation even in liver cancer, a correlation between MSH2 and a DNMT family involved in DNA methylation was confirmed in TCGA and ICGC data sets.

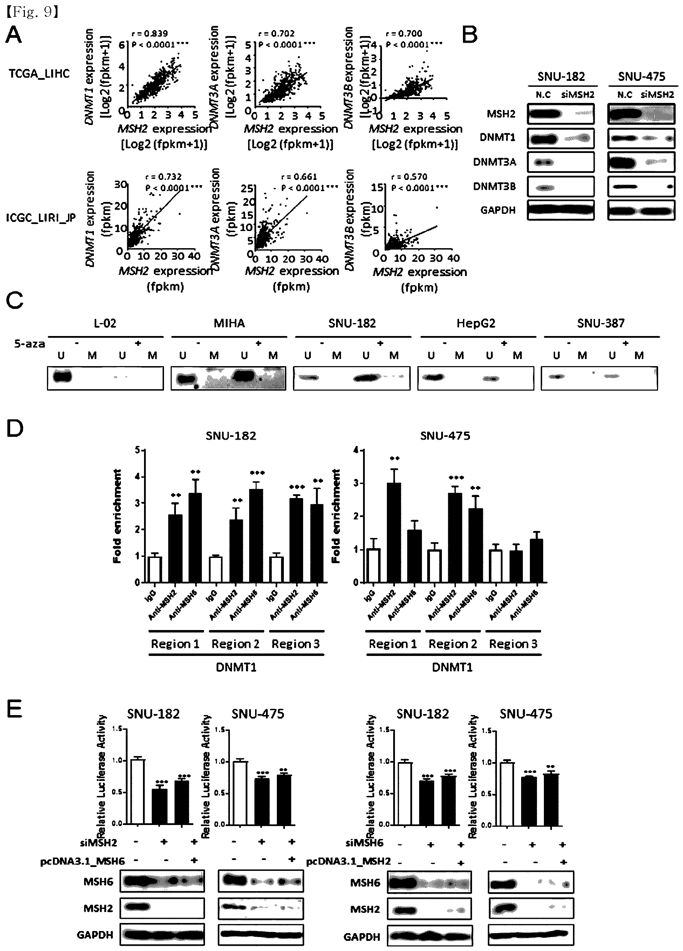

As a result, it was confirmed that in TCGA_LIHC, there was a significantly positive correlation between MSH2 and DNMT1, DNMT3A, and DNMT3B (r = 0.839, r = 0.702, r = 0.700, P < 0.0001), which showed the same results (r = 0.732, r = 0.661, r = 0.570, P < 0.0001) even in RNA seq data of ICGC_LIRI_JP, and thus the MSH2 had a strong positive correlation with the DNMT family of DNMT1, DNMA3A, and DNMT3B (FIG. 9A). Similarly, it was confirmed that when MSH2 was knocked down, protein expression of DNMT1, DNMT3A, and DNMT3B was reduced (FIG. 9B).

However, as the result of confirming the methylation of the promoter region of MSH2 through methylation specific PCR, methylation of MSH2 was not confirmed in HCC cell lines L-02, MIHA, SNU-182, HepG2, and SNU387 (FIG. 9C).

Accordingly, assuming that MSH2 regulated the expression of the DNMT family through another mechanism, ChIP assay (Pierce Agarose ChIP kit; Thermo Fisher Scientific, Waltham, MA) was performed to determine whether MSH2 bound to the promoters of DNMT1, DNMT3A, and DNMT3B. Cross-linked DNA was normalized with an IgG control and measured using an MSH2 antibody. Primers capable of identifying the promoter of the DNMT family were fabricated and binding was confirmed. As a result, it was confirmed that MSH2 bound only to the promoter region of DNMT 1 (FIG. 9D).

### 6-2. Confirmation of DNMT inhibitory activity of MSH2 inhibitor

Since it was confirmed that MSH2 bound to the promoter site of DNMT1, it could be inferred that MSH2 served as a transcription factor and regulated the expression of DNMT1. Accordingly, promoter luciferase assay was performed to confirm whether transcription of DNMT1 was regulated by MSH2. The promoter of DNMT1 was amplified by PCR from cDNA of cell lines SNU182 and SNU475 and then cloned into an Xho1Bgl2 site of a pGL3_basic vector (Promega, Madison, WI) to fabricate a reporter vector pGL3_basic_DNMT1. Thereafter, luciferase reporter analysis was performed by transfecting pGL3_basic_DNMT1 and si-MSH2 (SEQ ID NO: 1), and the results were compared with the results of a negative control siRNAtreated group (si-N.C). As a result, it was confirmed that the luciferase activity was significantly reduced in both cell lines SNU-182 and SNU-475 compared to the control group due to si-MSH2 treatment (FIG. 9E).

### Example 7. Verification of MSH6 with increased expression in liver cancer

### 7-1. Confirmation of MSH6 expression in HCC

To further analyze the expression of MSH6 in liver cancer, which ranked third in mortality among many carcinomas, TCGA, ICGC, GSE77314, and Catholic_mLIHC (GSE114564) data were analyzed. The experiment was performed in the same manner as in Example 3-1.

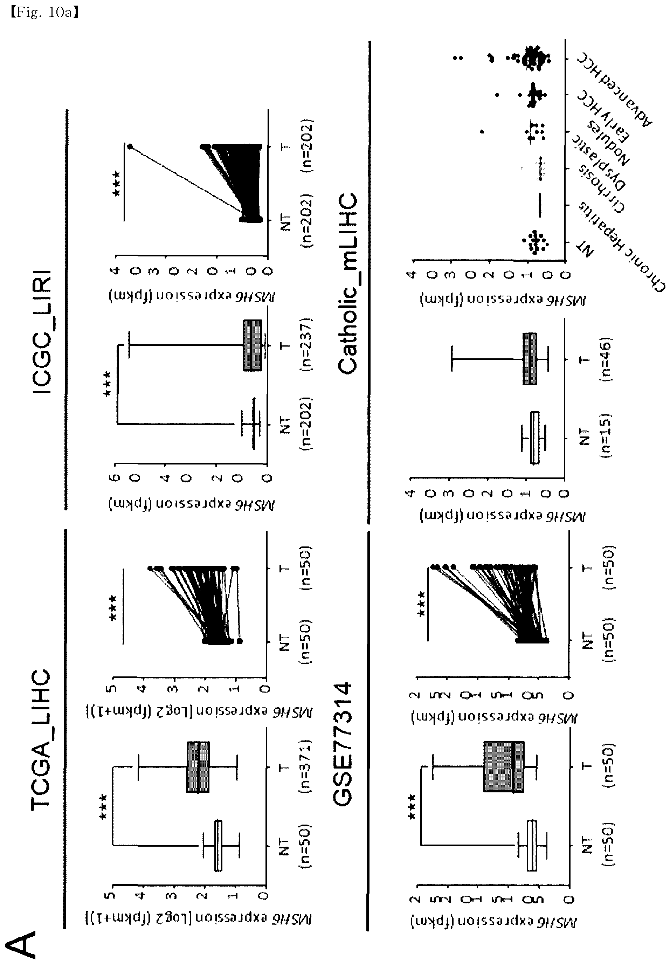

As a result, it was confirmed that the expression of MSH6 was increased in tumor compared to non-tumor, and the expression of MSH6 was also increased in paired samples of TCGA, ICGC, and GSE77314 data sets (FIG. 10A).

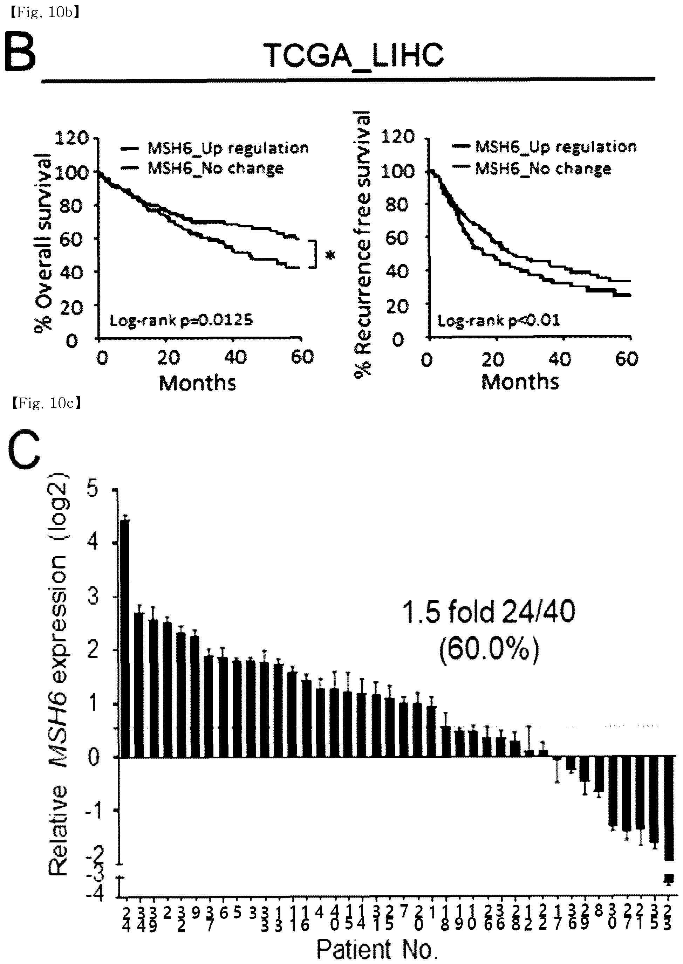

Subsequently, it was confirmed that when survival analysis (Kaplan-Meier survival analysis) was performed on the TCGA data set, patients with high expression of MSH6 had poor survival (FIG. 10B).

### 7-2. 40 matched-pair analysis

To identify the abnormal expression of MSH6 in HCC, 40 matched-pair analysis was performed using 40 matched-pairs (HCC tissue vs non-cancerous surrounding liver tissue) of HCC tissue and 13 HCC cell lines MIHA, L-02, Hep3B, Huh7, PLC/PRF/5, SNU182, SNU354, SNU368, SNU387, SNU398, SNU423, SNU449, and SNU475, and the experiment was performed in the same manner as Example 3-2.

As a result, it was confirmed through qRT-PCR that 24 (60.0%) of 40 HCC patients showed significant overexpression of MSH6 compared to non-cancerous liver tissue (FIG. 10C).

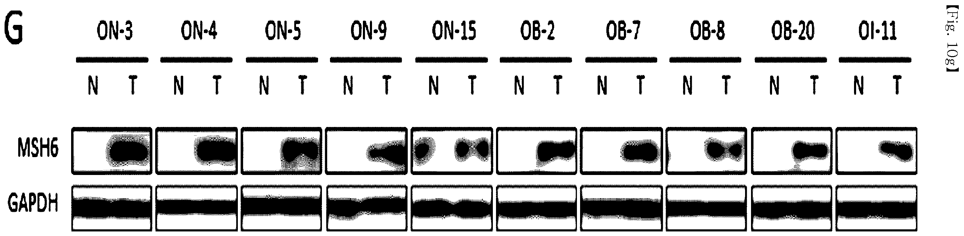

In addition, it was confirmed that when proteins were extracted from normal liver tissue and liver cancer tissue of a liver cancer patient distributed from the National Biobank and the expression of MSH6 protein was confirmed through Western blot, the expression of MSH6 protein was increased in tumor tissue (FIG. 10G).

### 7-3. Confirmation of MSH6 expression in liver cancer cell lines

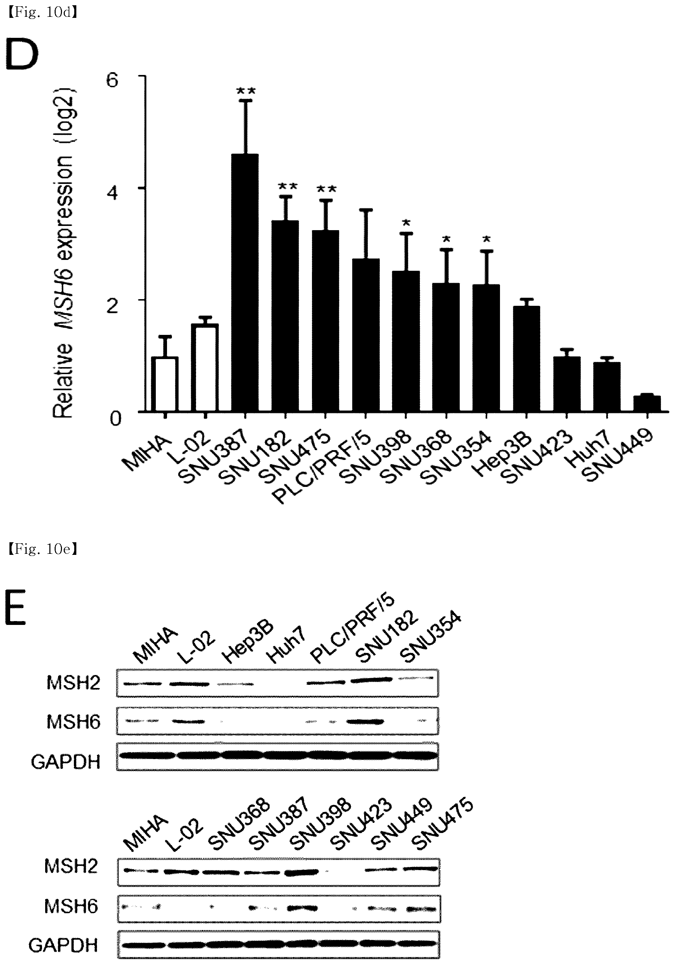

To confirm the MSH6 expression levels in various liver cancer cell lines, qRT-PCR and Western blot for MSH6 were performed. As a result, it was confirmed that the expression of MSH6 mRNA and protein was increased compared to normal hepatocytes (MIHA) (FIGS. 10D and 10E).

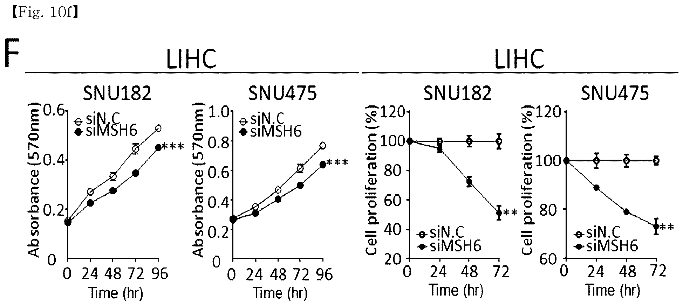

In addition, in order to confirm the effect of MSH6 inhibition in liver cancer with increased MSH6 expression, MSH6 was knocked down in liver cancer cell lines SNU182 and SNU475 with high MSH6 expression, and then MTT assay and BrdU assay were performed. As a result, it was confirmed that cell growth and cell proliferation decreased when MSH6 was inhibited in liver cancer cell lines with increased MSH6 (FIG. 10F).

### Example 8. Confirmation of characteristics of MSH6 in carcinogenic process

### 8-1. Confirmation of effect on tumor cell proliferation

To confirm the tumorigenic characteristics of MSH6 in liver cancer, clonogenic assay was performed on SNU182 and SNU475 transfected with si-MSH6, and the experiment was performed in the same manner as in Example 4-1.

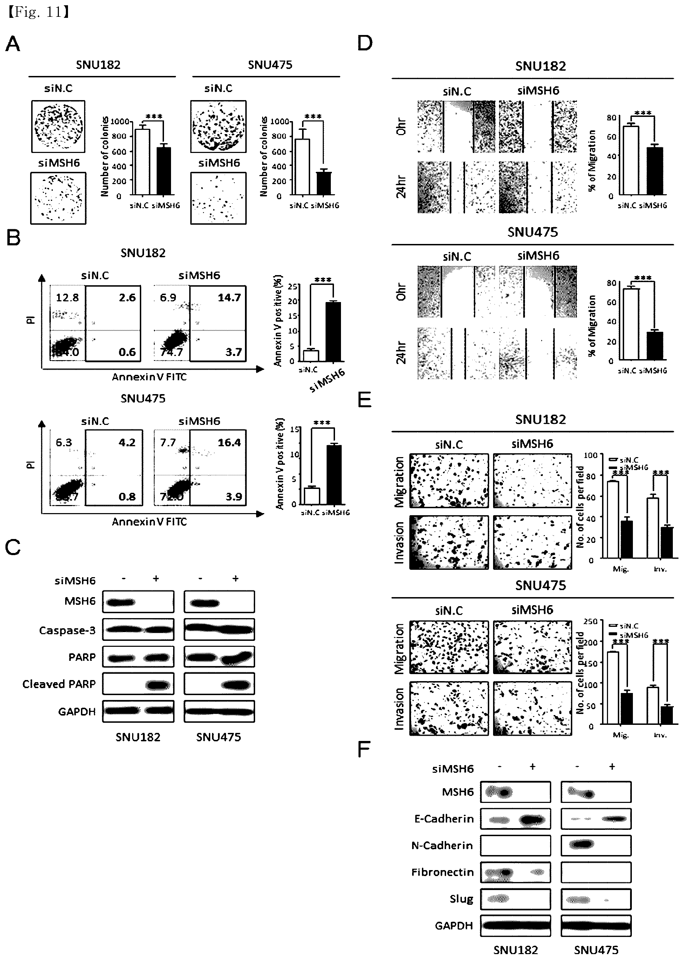

As a result, it was confirmed that colony formation was reduced when MSH6 was knocked down in liver cancer cell lines SNU182 and SNU475 with increased expression of MSH6 (FIG. 11A).

### 8-2. Confirmation of effect on apoptosis of tumor cells

Changes in apoptosis and cell cycle regulation caused by si-MSH6 transfection were confirmed, and the experiment was performed in the same manner as in Example 4-2.

As a result, it was confirmed through FACS analysis that apoptosis increased when MSH6 was knocked down (FIG. 11B), and similarly, as a protein expression analysis result, it was shown that PARP and caspase-3 decreased and cleaved PARP increased, and thus it was confirmed that PARP and caspase-3, which were involved in apoptosis, were activated (FIG. 11C).

### 8-3. Confirmation of effects on tumor cell migration and invasion

Since epithelial-to-mesenchymal transition (EMT) has been suggested as a key process in cancer progression along with cell growth, MSH6 was inhibited in HCC cells and a scratch wound healing assay was performed. The migration and invasion characteristics of cancer cells were confirmed through modified Boyden chamber assay (BD Biosciences, San Jose, CA) and transwell invasion assay. The experiment was performed in the same manner as in Example 4-3.

As a result, it was found that the wound healing effect was significantly reduced when MSH6 was knocked down in both SNU182 and SNU475 cells (FIG. 11D). In addition, when MSH6 was knocked down in both SNU182 and SNU475 cells, it was shown that the migration and invasion were significantly reduced (FIG. 11E).

To further verify the results, Western blot was performed on an EMT marker, and as a result, it was confirmed that N-Cadherin, Fibronectin, and Slug were decreased (FIG. 11F).

### 8-4. Confirmation of effect of changes in cell cycle regulation of tumor cells

When MSH6 was knocked down in liver cancer cell lines SNU182 and SNU475 with increased expression of MSH6, changes in cell cycle regulation were confirmed, and the experiment was performed in the same manner as in Example 4-4.

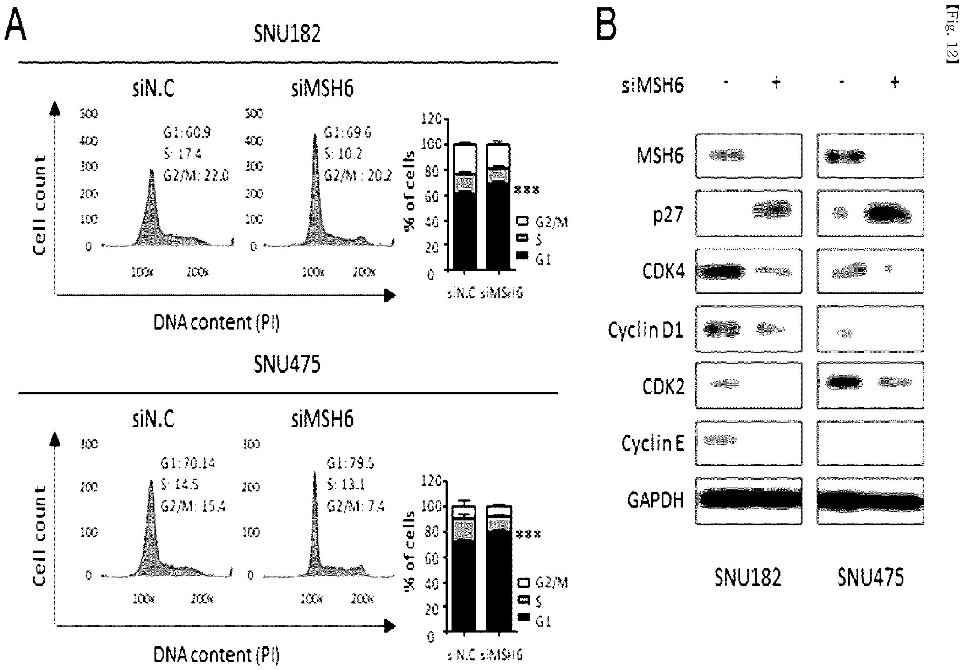

As a result, it was confirmed that a G1/S arrest was confirmed through FACS analysis when MSH6 was knocked down in liver cancer cell lines SNU182 and SNU475 with increased expression of MSH6 (FIG. 12A).

To further verify the results, Western blot was performed on cell cycle markers, and as a result, an increase in p27 and a decrease in CDK4, Cyclin D1, CDK2, and Cyclin E were confirmed (FIG. 12B).

Through the results, it could be seen that abnormal expression of MSH6 contributed to the anti-apoptotic and metastatic characteristics of HCC.

### Example 9. Confirmation of oncogene characteristics of MSH6

To confirm the oncogenic function of MSH6, MSH6 was overexpressed through transient transfection in a normal liver cell line (MIHA) and a liver cancer cell line (Huh7) with decreased expression of MSH6, and then MTT assay and BrdU assay were performed.

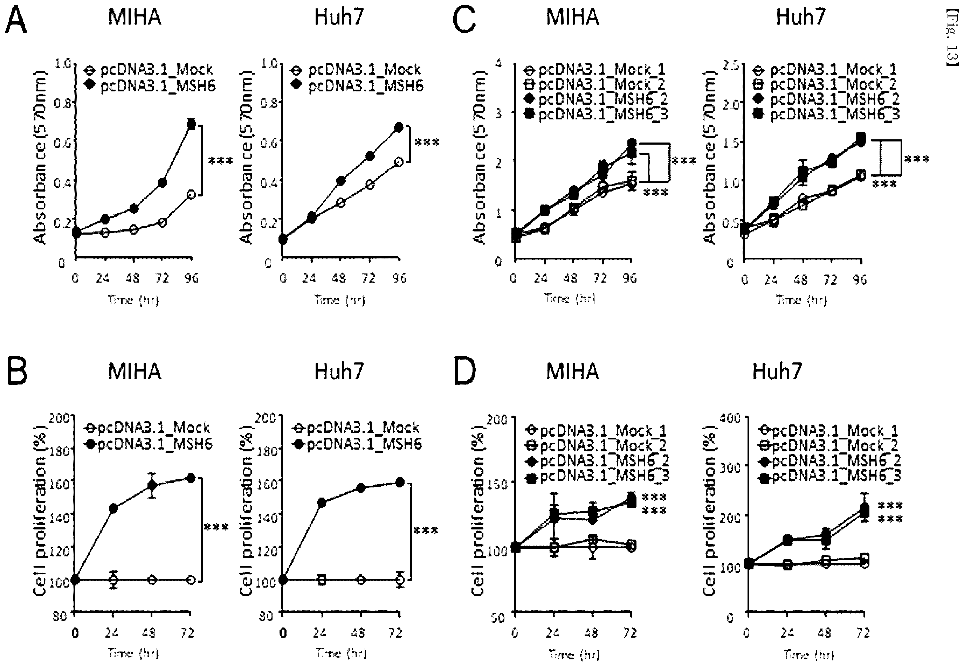

As a result, it was confirmed that cell growth and cell proliferation increased when MSH6 was overexpressed in the normal liver cell line and the liver cancer cell line with decreased MSH6 (FIG. 13A).

Next, a stable cell line was fabricated by continuously overexpressing MSH6 in the normal liver cell line (MIHA) and the liver cancer cell line (Huh7) with decreased expression of MSH6, and the fabrication of the cell line was performed in the same manner as in Example 5.

As a result, similarly to the results, it was confirmed that cell growth and cell proliferation increased (FIG. 13B).

### Example 10. Search of target molecule of MSH6

### 10-1. Search of target molecule of MSH6

In Lynch syndrome, MSH2 forming a hetero dimmer with MSH6 was known to be methylated in a promoter region. To confirm the methylation even in liver cancer, a correlation between MSH6 and a DNMT family involved in DNA methylation was confirmed in TCGA and ICGC data sets.

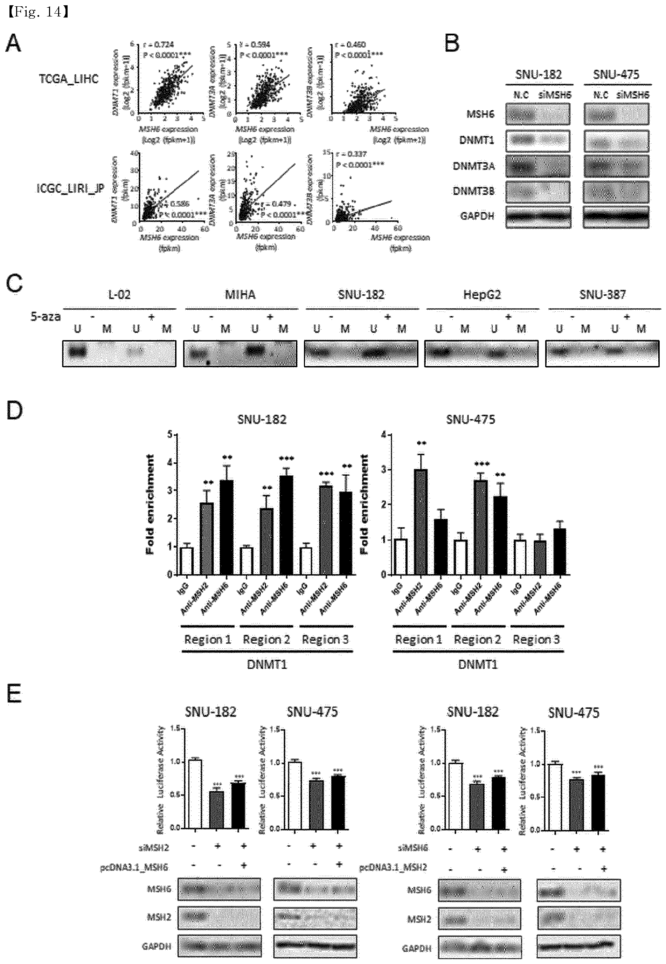

As a result, it was confirmed that in TCGA_LIHC, there was a significantly positive correlation between MSH6 and DNMT1, DNMT3A, and DNMT3B (r = 0.724, r = 0.594, r = 0.460, P < 0.0001), which showed the same results (r = 0.586, r = 0.479, r = 0.337, P < 0.0001) even in RNA seq data of ICGC_LIRI_JP, and thus the MSH6 had a strong positive correlation with the DNMT family of DNMT1, DNMA3A, and DNMT3B (FIG. 14A). Similarly, it was confirmed that when MSH6 was knocked down, protein expression of DNMT1, DNMT3A, and DNMT3B was reduced (FIG. 14B).

However, as the result of confirming the methylation of the promoter region of MSH6 through methylation specific PCR, methylation of MSH6 was not confirmed in HCC cell lines L-02, MIHA, SNU-182, HepG2, and SNU387 (FIG. 14C).

Accordingly, assuming that MSH6 regulated the expression of the DNMT family through another mechanism, ChIP assay (Pierce Agarose ChIP kit; Thermo Fisher Scientific, Waltham, MA) was performed to determine whether MSH6 bound to the promoters of DNMT1, DNMT3A, and DNMT3B. Cross-linked DNA was normalized with an IgG control and measured using an MSH2 antibody. Primers capable of identifying the promoter of the DNMT family were fabricated and binding was confirmed. As a result, it was confirmed that MSH6 bound only to the promoter region of DNMT 1 (FIG. 14D).

### 10-2. Confirmation of DNMT inhibitory activity of MSH6 inhibitor

Since it was confirmed that MSH6 bound to the promoter site of DNMT1, it could be inferred that MSH2 served as a transcription factor and regulated the expression of DNMT1. Accordingly, promoter luciferase assay was performed to confirm whether transcription of DNMT1 was regulated by MSH6, and the experiment was performed in the same manner as in Example 6-2.

As a result, it was confirmed that the luciferase activity was significantly reduced in both cell lines SNU-182 and SNU-475 compared to the control group due to si-MSH2 treatment (FIG. 14E).

### Example 11. Confirmation of correlation of MSH2/MSH6-DNMT1-KLF4 in liver cancer

It was known that DNMT1 methylated a promoter of KLF4 to inhibit the expression. Accordingly, to confirm the correlation between DNMT1 and KLF4 even in liver cancer, MSH2 or MSH6 was overexpressed or suppressed and then KLF4 methylation specific PCR was performed.

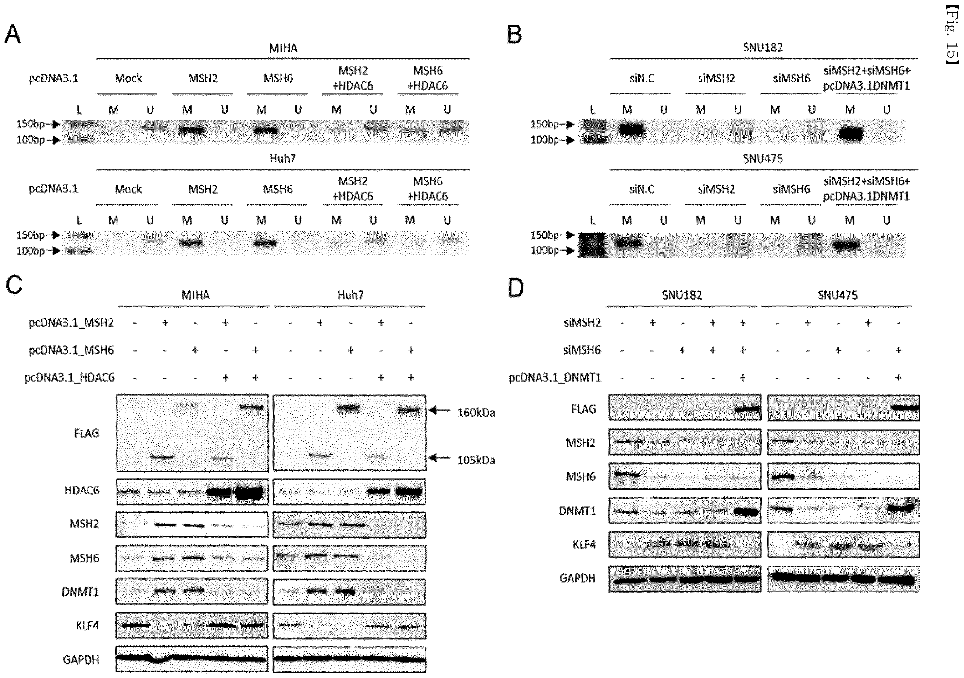

As a result, it was confirmed that when MSH2 or MSH6 was overexpressed, DNMT1 increased and the promoter methylation of KLF4 increased (FIG. 15A). On the contrary, when MSH2 or MSH6 was knocked down, MSH2 or MSH6 decreased, DNMT1 expression decreased, and the promoter methylation of KLF4 decreased (FIG. 15B).

In addition, in order to confirm changes in the expression of DNMT1 and KLF4 due to MSH2 and MSH6 at the protein level, MSH2 or MSH6 was overexpressed or suppressed, and then Western blot was performed for MSH2, MSH6, DNMT1, and KLF4 to evaluate the protein expression level.

As a result, as illustrated in FIGS. 15A and 15B, it was confirmed that when MSH2 or MSH6 was overexpressed, the expression of DNMT1 increased, and the promoter methylation of KLF4 increased, and thus the protein expression of KLF4 increased (FIG. 15C). On the contrary, when MSH2 was knocked down, MSH2 or MSH6 was decreased, and thus the expression of DNMT1 decreased, and KLF4 promoter methylation was decreased, and thus the protein expression of KLF4 increased (FIG. 15D).

### Example 12. Confirmation of correlation of HDAC2/KLF6-HDAC6-MSH2/MSH6 in liver cancer

### 12-1. Changes in expression of MSH2 or MSH6 depending on HDAC6 levels

To determine whether the expression of MSH2 or MSH6 was decreased while being ubiquitinated by HDAC6 in liver cancer, in TCGA, ICGC, and GSE77314 data, the expression of MSH6 and MSH2 and MSH6 serving as a dimer was confirmed due to changes in the expression of HDAC6.

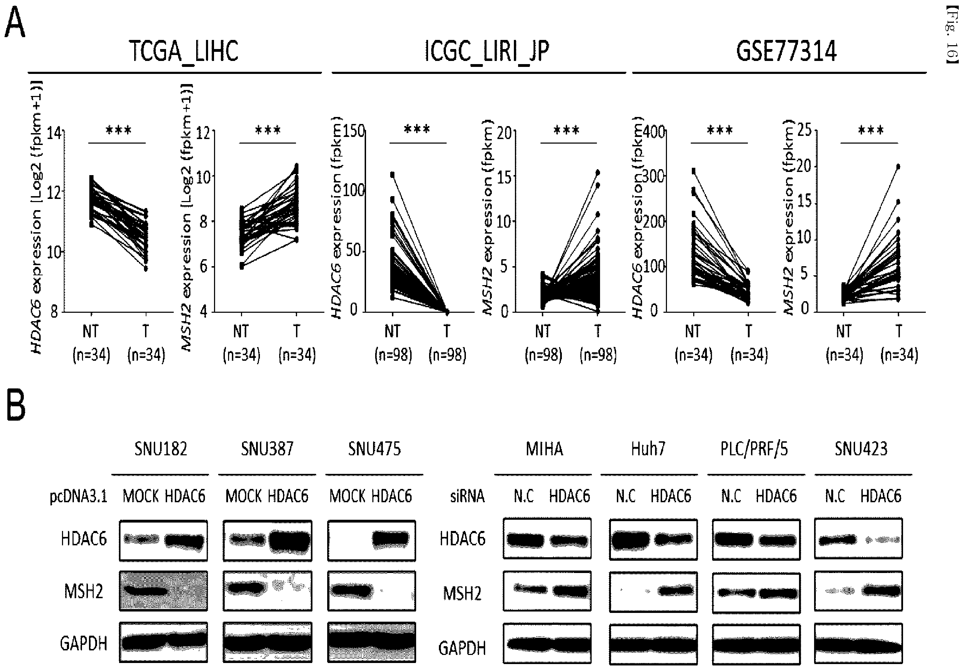

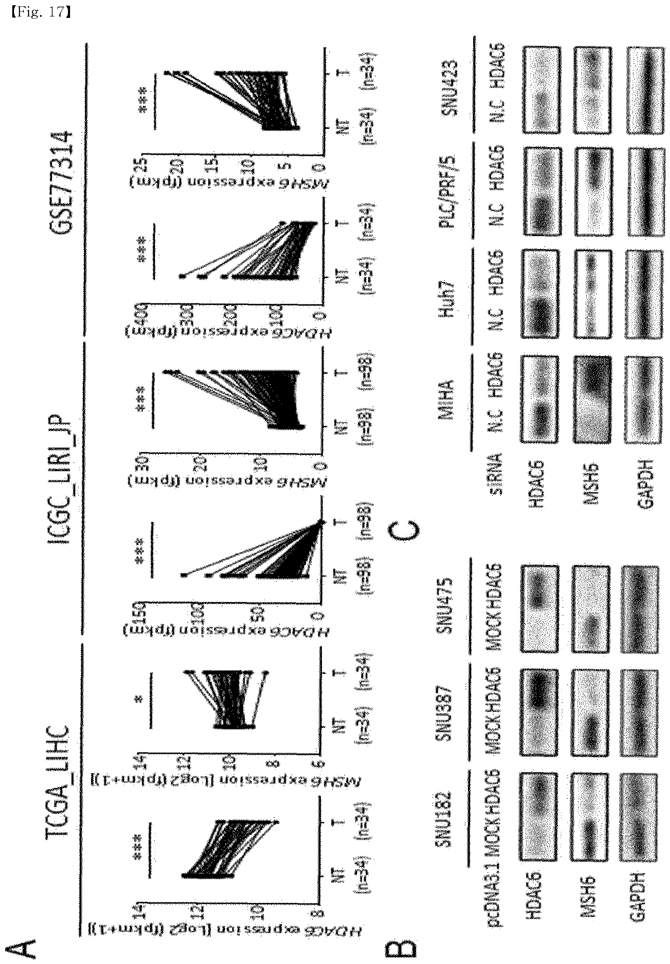

As a result, it was confirmed that in sub-groups (TGCA n = 34, ICGC n = 98, GSE77314 n = 34) where HDAC6 expression was decreased, the expression of MSH2 and MSH6 was increased in tumor (FIGS. 16A and 17A).

In addition, HDAC6 was overexpressed or suppressed in HCC cell lines SNU182, SNU387, and SNU475, and then Western blot for HDAC6 and MSH2 was performed to evaluate protein expression levels.

As a result, it was confirmed that when HDAC6 was overexpressed, the expression of MSH2 protein and MSH6 protein decreased, and conversely, when HDAC6 was knocked down, the expression of MSH2 protein increased (FIGS. 16B, 16C and 17B, 17C).

These results mean that HDAC6-MSH2 and HDAC6-MSH6 have a negative correlation and that the expression of MSH2 and MSH6 is regulated depending on the level of HDAC6.

### 12-2. Confirmation of HDAC2/KLF6-HDAC6 correlation

It was known that HDAC6 had a Gcbox in the promoter region, and the Gcbox was a region where a transcription factor can bind and HDAC2 and KLF6 can bind. Accordingly, the expression levels of HDAC2, HDAC6, and KLF6 genes were confirmed in hepatocellular carcinoma (HCC) patients and an HDAC2-HDAC6, KLF6-HDAC6, and KLF6-HDAC2 correlation was analyzed.

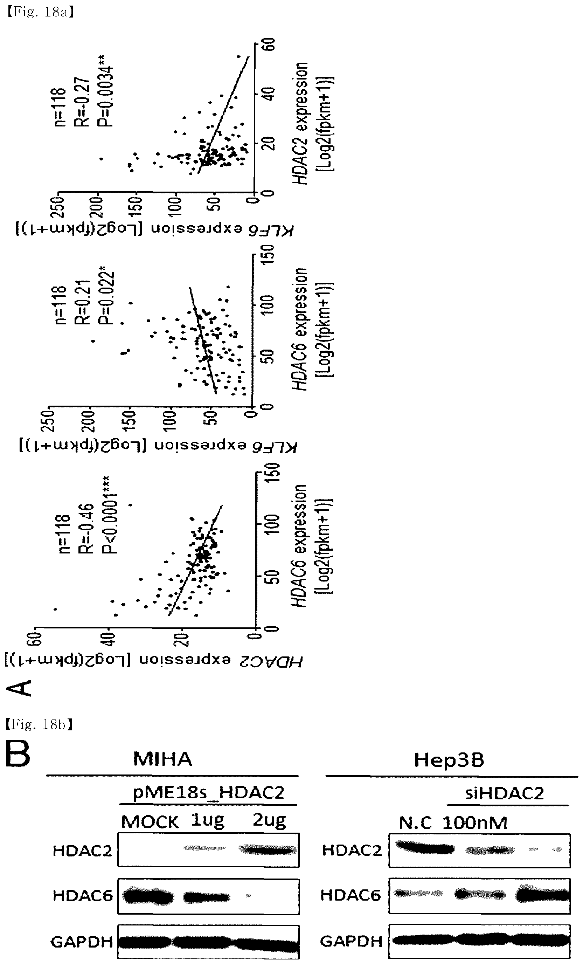

As a result, it was confirmed that HDAC6 and HDAC2, KLF6 and HDAC2 had a negative correlation, and HDAC6 and KLF6 had a positive correlation (FIG. 18A).

To further verify an HDAC2-HDAC6 and KLF6-HDAC6 correlation, in a normal liver cell line (MIHA) with low expression of HDAC2 and high expression of HDAC6 and KLF6, and a liver cancer cell line (Hep3B) with high expression of HDAC2 and low expression of HDAC6 and KLF6, changes in protein and mRNA expression levels were confirmed by controlling HDAC2 or KLF6 expression.

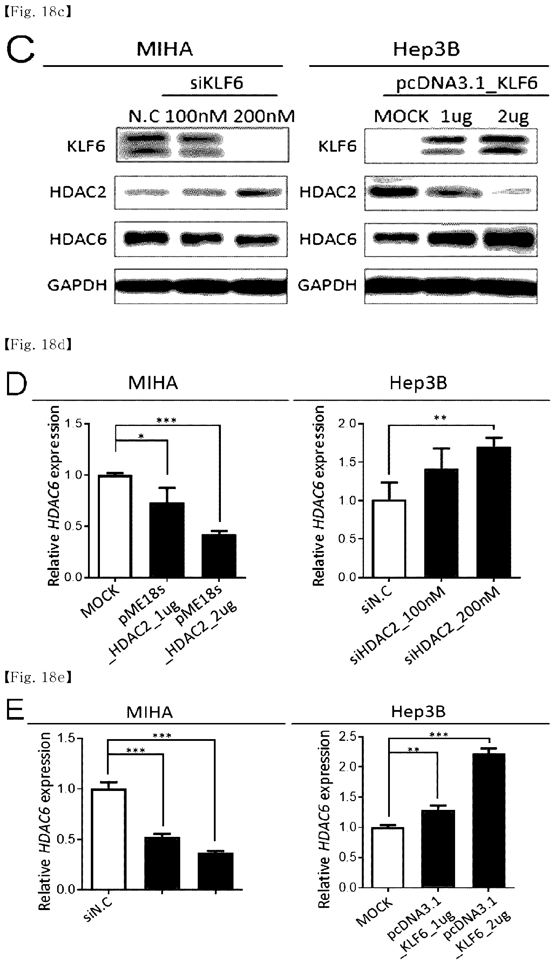

As a result, it was shown that when HDAC2 was overexpressed, the expression of HDAC6 decreased, and when HDAC2 was knocked down, the expression of HDAC6 increased (FIGS. 18B and 18D). In addition, it was shown that when KLF6 was overexpressed, the expression of HDAC2 decreased and the expression of HDAC6 increased, and when KLF6 was knocked down, the expression of HDAC2 increased and the expression of HDAC6 decreased (FIGS. 18C and 18E).

### 12-3. Confirmation of HDAC6 inhibitory activity of KLF6 or HDAC2 inhibitor

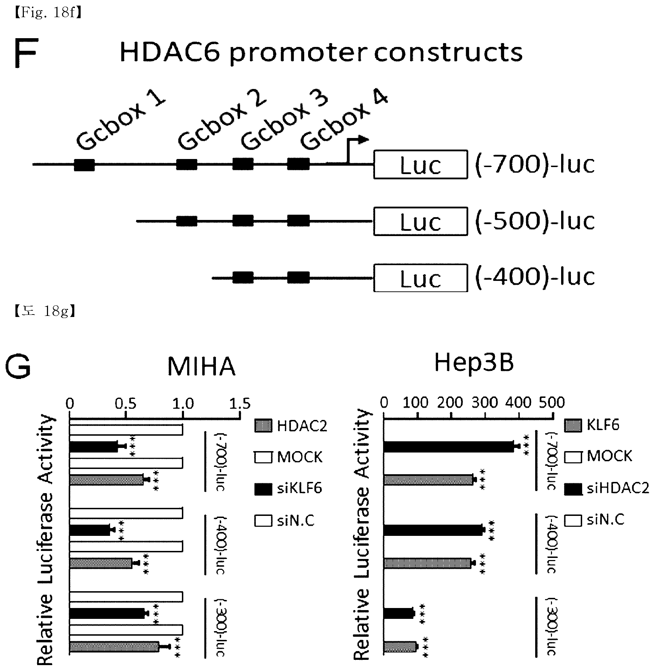

Promoter luciferase assay was performed to confirm whether transcription of HDAC6 was regulated by KLF6 or HDAC2. FIG. 18F simply shows a vector structure for promoter luciferase assay of HDAC6.

As a result, it was confirmed that the luciferase activity of the promoter vector decreased when KLF6 was knocked down or HDAC2 was overexpressed in an MIHA cell line as the normal cells, and it was confirmed that the luciferase activity of the promoter vector increased when KLF6 was overexpressed or HDAC2 was knocked down in a Hep3B cell line as liver cancer cells (FIG. 18G).

### 12-4. Confirmation of competitive binding of HDAC2 and KLF6

To confirm whether HDAC2 and KLF6 competitively bound to the promoter of HDAC6, HDAC2 was overexpressed and KLF6 was knocked down in a normal liver cell line MIHA with low expression of HDAC2 and high expression of HDAC6 and KLF6, and then HDAC2 and KLF6 were subjected to immunoprecipitation, respectively, and the amount of the HDAC6 promoter bound to HDAC2 and KLF6 was relatively calculated.

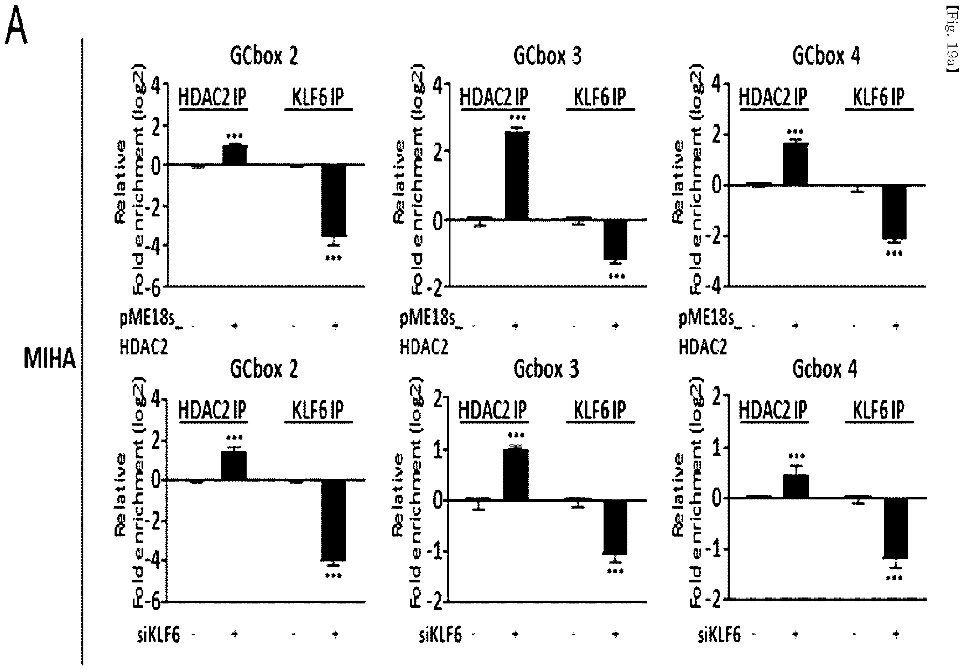

As a result, it was confirmed that when HDAC2 was overexpressed or KLF6 was knocked down commonly in three Gcbox regions present in the promoter of HDAC6, respectively, the amount of HDAC6 promoter binding to HDAC2 increased, and the amount of HDAC6 promoter binding to KLF6 decreased (FIG. 19A).

Subsequently, in a liver cancer cell line Hep3B with high expression of HDAC2 and low expression of HDAC6 and KLF6, KLF6 was overexpressed and HDAC2 was knocked down and then HDAC2 and KLF6 were subjected to immunoprecipitation, respectively, and the amount of HDAC6 promoter binding to HDAC2 and KLF6 was relatively calculated.

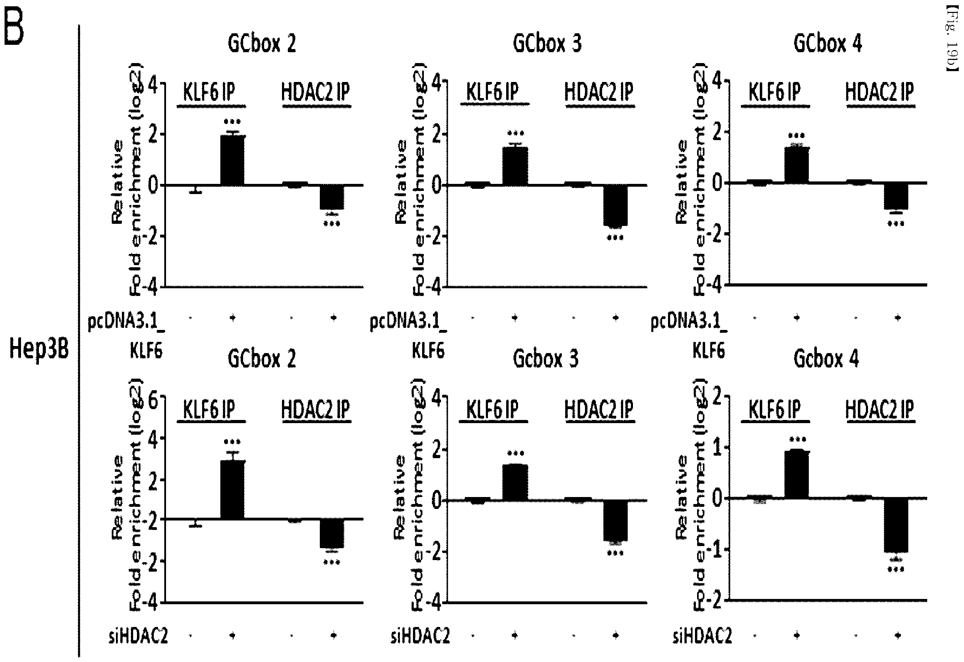

As a result, contrary to the results in FIG. 19A, it was confirmed that when HDAC2 was knocked down or KLF6 was overexpressed, the amount of HDAC6 promoter binding to HDAC2 decreased, and the amount of HDAC6 promoter binding to KLF6 increased (FIG. 19B).

These results mean that as the amount of HDAC6 promoter binding was changed when the expression of KLF6 and HDAC2 was controlled, respectively, KLF6 and HDAC2 competitively bound to the HDAC6 promoter.

In addition, through the previous promoter luciferase assay result, since it was confirmed that KLF6 and HDAC2 regulated the expression of HDAC6, KLF6 and HDAC2 competitively bound to the promoter of HDAC6 to regulate the expression of HDAC6.

### Example 13. Confirmation of cancer-prevention effect of HDAC6-MSH2-DNMT1-KLF4 signaling activation

### 13-1. In vivo confirmation of cancer-prevention effect of HDAC6-MSH2-DNMT1-KLF4 signaling activation

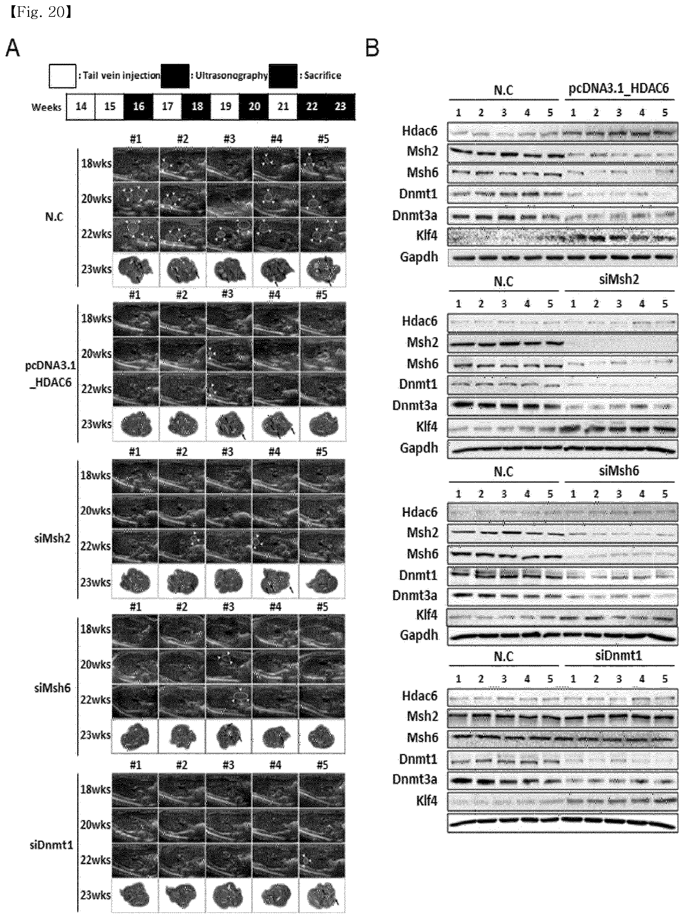

In order to confirm the contents *in vivo,* H*-ras* transgenic mice (Ras-Tg mice) (Laboratory of Human Genomics, Korea Research Institute of Bioscience and Biotechnology, Daejeon, Korea) were liver-specifically transferred weekly from 14 weeks of age with 50 ug of pcDNA3.1_HDAC6 (inj ected intravenously with Turbofect in vivo Transfection Reagent (Invitrogen, Carlsbad, CA)), si-MSH2 (SEQ ID NO: 1), si-MSH6 (SEQ ID NO: 2), or si-DNMT1 at 0.25 mg/kg (intravenously with Invivofectamine 3.0 (Invitrogen)). Thereafter, ultrasound images were taken with an ultrasound machine (Philips, Amsterdam, Nederland) at 16, 18, 20, and 22 weeks of age, and livers were obtained at 23 weeks of age to confirm HCC. HCC in the liver of negative control (N.C) mice was detected by ultrasound from 16 weeks of age, and all five of five mice were developed to large and multiple HCC. However, in mice overexpressing HDAC6, HCC was detected at 20 weeks of age (1 mouse), and small HCC was developed in two to three of five mice in an MSH2 inhibition group (si-MSH2) and a DNMT1 inhibition group (si-DNMT1) (FIG. 20A).

In addition, as the Western blot analysis result, it was confirmed that DNMT family expression was decreased and KLF4 expression was increased in the adjacent normal liver of the si-MSH2 group (FIG. 20B).

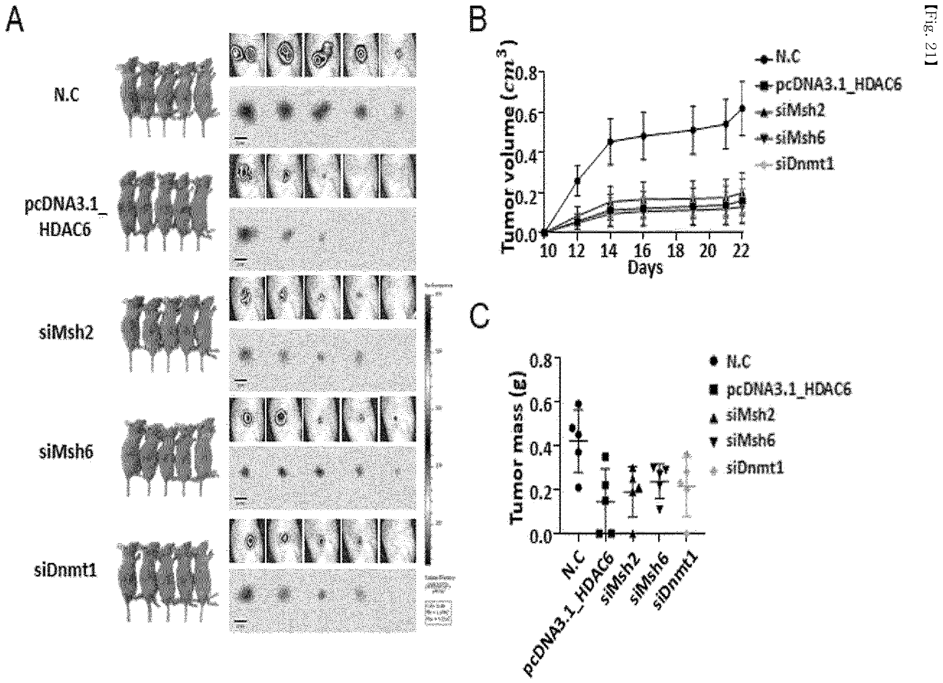

This was also confirmed in a Mouse Xenograft model (FIG. 21A). In a Xenograft model, a mouse HCC cell line (Hepa1-6) was injected subcutaneously at 1 × 10⁶ into the flank of an immunosuppressed nude mouse together with 20% matrigel. After injection, the size thereof was checked at two-day intervals (FIG. 21B), and its weight was checked 2 weeks later (FIG. 21C).

As a result, it was confirmed that in the si-MSH2 group, the tumor growth was significantly reduced compared to the control group (N.C).

## Claims

1. A pharmaceutical composition for preventing or treating cancer comprising as an active ingredient an inhibitor for an MutS homolog2 (MSH2) gene or protein; or an inhibitor for an MutS homolog6 (MSH6) gene or protein.

2. The pharmaceutical composition for preventing or treating cancer of claim 1, wherein the inhibitor is selected from the group consisting of siRNA, shRNA, miRNA, antisense oligonucleotide, aptamers, antibodies, and combinations thereof, which are specific for MSH2 or MSH6.

3. The pharmaceutical composition for preventing or treating cancer of claim 2, wherein the siRNA specific for the MSH2 is represented by a base sequence set forth in SEQ ID NO: 1.

4. The pharmaceutical composition for preventing or treating cancer of claim 2, wherein the siRNA specific for the MSH6 is represented by a base sequence set forth in SEQ ID NO: 2.

5. The pharmaceutical composition for preventing or treating cancer of claim 1, wherein the cancer is selected from the group consisting of liver cancer, lung cancer, head and neck cancer, colon cancer, sarcoma, stomach cancer, and gallbladder cancer.

6. The pharmaceutical composition for preventing or treating cancer of claim 1, wherein the cancer is cancer of a patient with inhibited expression of Kruppel-like factor 6 (KLF6), Histone deacetylase 6 (HDAC6), or Kruppel-like factor 4 (KLF4), or increased expression of Histone deacetylase 2 (HDAC2), mutS homolog2 (MSH2), or DNA (cytosine-5)-Methyltransferase 1 (DNMT1).

7. The pharmaceutical composition for preventing or treating cancer of claim 1, wherein the composition inhibits expression of DNMT1 or increases expression of KLF4.

8. The pharmaceutical composition for preventing or treating cancer of claim 1, wherein the composition inhibits growth, proliferation, migration and invasion of tumor cells.

9. A composition for diagnosing any one cancer selected from the group consisting of liver cancer, lung cancer, head and neck cancer, colon cancer, sarcoma, stomach cancer, and gallbladder cancer, comprising a preparation for measuring a level of an MSH2 or MSH6 gene, its protein, or a peptide constituting the protein.

10. The composition for diagnosing cancer of claim 9, wherein the preparation for measuring the gene level includes a primer or probe that specifically binds to the gene.

11. The composition for diagnosing cancer of claim 9, wherein the preparation for measuring the protein level includes any one selected from the group consisting of antibodies, antisense RNA, aptamers, and compounds, which are specific for the protein or fragment peptide.

12. A method for screening a substance for prevention or treatment of any one cancer selected from the group consisting of liver cancer, lung cancer, head and neck cancer, colon cancer, sarcoma, stomach cancer, and gallbladder cancer, the method comprising:
(a) contacting a sample to be analyzed with cells including an MSH2 gene or its protein; or an MSH6 gene or its protein;
(b) measuring an expression level of the MSH2 or MSH6 gene, a protein expression level, or a protein activity; and
(c) determining the sample as a substance for preventing or treating cancer when the expression level of the MSH2 or MSH6 gene, the protein expression level, or the protein activity is reduced as a result of the measurement in step (b).

13. A method for treating liver cancer comprising administering the pharmaceutical composition of claim 1 to a subject.
